# EUROPÄISCHE PATENTSCHRIFT

(11) **EP 1 458 866 B1**
(45) Veröffentlichungstag und Bekanntmachung des Hinweises auf die Patenterteilung: **26.03.2008**
(21) Anmeldenummer: 02805347.8
(22) Anmeldetag: 20.12.2002
(51) Int. Cl.: C12N 15/12, C07K 14/435, C12N 15/62, A61K 38/17, A61P 7/02

(54) **MODIFIZIERTE TRIDEGINE, IHRE HERSTELLUNG UND VERWENDUNG ALS TRANSGLUTAMINASE-INHIBITOREN**
MODIFIED TRIDEGINS, PRODUCTION AND USE THEREOF AS TRANSGLUTAMINASE INHIBITORS
TRIDEGINES MODIFIEES, LEUR PRODUCTION ET LEUR UTILISATION COMME INHIBITEURS DE TRANSGLUTAMINASE

(30) Priorität: 21.12.2001 DE 10163333; 12.12.2002 DE 10258159
(43) Veröffentlichungstag der Anmeldung: 22.09.2004
(73) Patentinhaber: Curacyte AG, 81675 München (DE)
(72) Erfinder: GIERSIEFEN, Helmut, 82319 Starnberg (DE); STÖCKEL, Johannes, 81369 München (DE); PAMP, Tanja, 81373 München (DE); OHLMANN, Marion, 81927 München (DE)
(74) Vertreter: Bösl, Raphael Konrad
(86) Internationale Anmeldenummer: PCT/EP2002/014684
(87) Internationale Veröffentlichungsnummer: WO 2003/054194

(56) Entgegenhaltungen:
- WO-A-00/49039
- WO-A-96/34890
- US-B1- 6 265 187
- WALLIS, R.B. ET AL.: "Reduction of plasma clot stability by a novel factor XIIIa inhibitor from the Giant Amazon Leech, Haementeria ghilianii" BLOOD COAGULATION & FIBRINOLYSIS, Bd. 8, Nr. 5, Juli 1997 (1997-07), Seiten 291-295, XP008015989

### SEQUENCE LISTING

<110> Curacyte AG
<120> Polypeptid-Inhibitoren von Transglutaminasen
<130> C36861PC
<140> PCT/EP 02/14684
   <141> 2002-12-20
<150> DE 1016333.5
   <151> 2001-12-21
<150> DE 10258159.2
   <151> 2002-12-12
<160> 92
<170> PatentIn version 3.1
<210> 1
   <211> 68
   <212> PRT
   <213> Künstliche Sequenz
<220>
   <223> Wildtyp Tridegin
<400> 1
   Met Lys Leu Leu Pro Cys Lys Glu Trp His Gln Gly Ile Pro Asn Pro
   1               5                   10                  15
   Arg Cys Trp Cys Gly Ala Asp Leu Glu Cys Ala Gln Asp Gln Tyr Cys
               20                  25                  30
   Ala Phe Ile Pro Gln Cys Arg Pro Arg Ser Glu Leu Ile Lys Pro Met
           35                  40                  45
   Asp Asp Ile Tyr Gln Arg Pro Val Glu Phe Pro Asn Leu Pro Leu Lys
       50                  55                  60
   Pro Arg Glu Glu
   65
<210> 2
   <211> 20
   <212> PRT
   <213> Künstliche Sequenz
<220>
   <223> Peptid 1
<400> 2
   Met Lys Leu Leu Pro Cys Lys Glu Trp His Gln Gly Ile Pro Asn Pro
   1               5                   10                  15
   Arg Cys Trp Cys
               20
<210> 3
   <211> 20
   <212> PRT
   <213> Künstliche Sequenz
<220>
   <223> Peptid 2
<400> 3
   Leu Leu Pro Cys Lys Glu Trp His Gln Gly Ile Pro Asn Pro Arg Cys
   1               5                   10                  15
   Trp Cys Gly Ala
               20
<210> 4
   <211> 20
   <212> PRT
   <213> Künstliche Sequenz
<220>
   <223> Peptid 3
<400> 4
   Pro Cys Lys Glu Trp His Gln Gly Ile Pro Asn Pro Arg Cys Trp Cys
   1               5                   10                  15
   Gly Ala Asp Leu
               20
<210> 5
   <211> 20
   <212> PRT
   <213> Künstliche Sequenz
<220>
   <223> Peptid 4
<400> 5
   Lys Glu Trp His Gln Gly Ile Pro Asn Pro Arg Cys Trp Cys Gly Ala
   1               5                   10                  15
   Asp Leu Glu Cys
               20
<210> 6
   <211> 20
   <212> PRT
   <213> Künstliche Sequenz
<220>
   <223> Peptid 5
<400> 6
   Trp His Gln Gly Ile Pro Asn Pro Arg Cys Trp Cys Gly Ala Asp Leu
   1 5 10 15
   Glu Cys Ala Gln
   20
<210> 7
   <211> 20
   <212> PRT
   <213> Künstliche Sequenz
<220>
   <223> Peptid 6
<400> 7
   Gln Gly Ile Pro Asn Pro Arg Cys Trp Cys Gly Ala Asp Leu Glu Cys
   1 5 10 15
   Ala Gln Asp Gln
   20
<210> 8
   <211> 20
   <212> PRT
   <213> Künstliche Sequenz
<220>
   <223> Peptid 7
<400> 8
   Ile Pro Asn Pro Arg Cys Trp Cys Gly Ala Asp Leu Glu Cys Ala Gln
   1 5 10 15
   Asp Gln Tyr Cys
   20
<210> 9
   <211> 20
   <212> PRT
   <213> Künstliche Sequenz
<220>
   <223> Peptid 8
<400> 9
   Asn Pro Arg Cys Trp Cys Gly Ala Asp Leu Glu Cys Ala Gln Asp Gln
   1 5 10 15
   Tyr Cys Ala Phe
   20
<210> 10
   <211> 20
   <212> PRT
   <213> Künstliche Sequenz
<220>
   <223> Peptid 9
<400> 10
   Arg Cys Trp Cys Gly Ala Asp Leu Glu Cys Ala Gln Asp Gln Tyr Cys
   1 5 10 15
   Ala Phe Ile Pro
   20
<210> 11
   <211> 20
   <212> PRT
   <213> Künstliche Sequenz
<220>
   <223> Peptid 10
<400> 11
   Trp Cys Gly Ala Asp Leu Glu Cys Ala Gln Asp Gln Tyr Cys Ala Phe
   1 5 10 15
   Glu Pro Gln Cys
   20
<210> 12
   <211> 20
   <212> PRT
   <213> Künstliche Sequenz
<220>
   <223> Peptid 11
<400> 12
   Gly Ala Asp Leu Glu Cys Ala Gln Asp Gln Tyr Cys Ala Phe Ile Pro
   1 5 10 15
   Gln Cys Arg Pro
   20
<210> 13
   <211> 20
   <212> PRT
   <213> Künstliche Sequenz
<220>
   <223> Peptid 12
<400> 13
   Asp Leu Glu Cys Ala Gln Asp Gln Tyr Cys Ala Phe Ile Pro Gln Cys
   1 5 10 15
   Arg Pro Arg Ser
   20
<210> 14
   <211> 20
   <212> PRT
   <213> Künstliche Sequenz
<220>
   <223> Peptid 13
<400> 14
   Glu Cys Ala Gln Asp Gln Tyr Cys Ala Phe Ile Pro Gln Cys Arg Pro
   1 5 10 15
   Arg Ser Glu Leu
   20
<210> 15
   <211> 20
   <212> PRT
   <213> Künstliche Sequenz
<220>
   <223> Peptid 14
<400> 15
   Ala Gln Asp Gln Tyr Cys Ala Phe Ile Pro Gln Cys Arg Pro Arg Ser
   1 5 10 15
   Glu Leu Ile Lys
   20
<210> 16
   <211> 20
   <212> PRT
   <213> Künstliche Sequenz
<220>
   <223> Peptid 15
<400> 16
   Asp Gln Tyr Cys Ala Phe Ile Pro Gln Cys Arg Pro Arg Ser Glu Leu
   1 5 10 15
   Ile Lys Pro Met
   20
<210> 17
   <211> 20
   <212> PRT
   <213> Künstliche Sequenz
<220>
   <223> Peptid 16
<400> 17
   Tyr Cys Ala Phe Glu Pro Gln Cys Arg Pro Arg Ser Glu Leu Ile Lys
   1 5 10 15
   Pro Met Asp Asp
   20
<210> 18
   <211> 20
   <212> PRT
   <213> Künstliche Sequenz
<220>
   <223> Peptid 17
<400> 18
   Ala Phe Ile Pro Gln Cys Arg Pro Arg Ser Glu Leu Ile Lys Pro Met
   1               5                   10                  15
   Asp Asp Ile Tyr
               20
<210> 19
   <211> 20
   <212> PRT
   <213> Künstliche Sequenz
<220>
   <223> Peptid 18
<400> 19
   Ile Pro Gln Cys Arg Pro Arg Ser Glu Leu Ile Lys Pro Met Asp Asp
   1               5                   10                  15
   Ile Tyr Gln Arg
               20
<210> 20
   <211> 20
   <212> PRT
   <213> Künstliche Sequenz
<220>
   <223> Peptid 19
<400> 20
   Gln Cys Arg Pro Arg Ser Glu Leu Ile Lys Pro Met Asp Asp Ile Tyr
   1               5                   10                  15
   Gln Arg Pro Val
               20
<210> 21
   <211> 20
   <212> PRT
   <213> Künstliche Sequenz
<220>
   <223> Peptid 20
<400> 21
   Arg Pro Arg Ser Glu Leu Ile Lys Pro Met Asp Asp Ile Tyr Gln Arg
   1 5 10 15
   Pro Val Glu Phe
   20
<210> 22
   <211> 20
   <212> PRT
   <213> Künstliche Sequenz
<220>
   <223> Peptid 21
<400> 22
   Arg Ser Glu Leu Ile Lys Pro Met Asp Asp Ile Tyr Gln Arg Pro Val
   1 5 10 15
   Glu Phe Pro Asn
   20
<210> 23
   <211> 20
   <212> PRT
   <213> Künstliche Sequenz
<220>
   <223> Peptid 22
<400> 23
   Glu Leu Ile Lys Pro Met Asp Asp Ile Tyr Gln Arg Pro Val Glu Phe
   1 5 10 15
   Pro Asn Leu Pro
   20
<210> 24
   <211> 20
   <212> PRT
   <213> Künstliche Sequenz
<220>
   <223> Peptid 23
<400> 24
   Ile Lys Pro Met Asp Asp Ile Tyr Gln Arg Pro Val Glu Phe Pro Asn
   1               5                   10                  15
   Leu Pro Leu Lys
               20
<210> 25
   <211> 20
   <212> PRT
   <213> Künstliche Sequenz
<220>
   <223> Peptid 24
<400> 25
   Pro Met Asp Asp Ile Tyr Gln Arg Pro Val Glu Phe Pro Asn Leu Pro
   1               5                   10                  15
   Leu Lys Pro Arg
               20
<210> 26
   <211> 20
   <212> PRT
   <213> Künstliche Sequenz
<220>
   <223> Peptid 25
<400> 26
   Asp Asp Ile Tyr Gln Arg Pro Val Glu Phe Pro Asn Leu Pro Leu Lys
   1               5                   10                  15
   Pro Arg Glu Glu
               20
<210> 27
   <211> 54
   <212> DNA
   <213> Künstliche Sequenz
<220>
   <223> Mutante D50A
<400> 27
   ctcgactgga cgttggtaaa tagcatccat aggtttaatc agttctgaac gtgg 54
<210> 28
   <211> 43
   <212> DNA
   <213> Künstliche Sequenz
<220>
   <223> Mutante F34A
<400> 28
   gtggacgaca ttgaggaatg gcggcacagt attggtcttg tgc 43
<210> 29
   <211> 54
   <212> DNA
   <213> Künstliche Sequenz
<220>
   <223> Mutante F58A
<400> 29
   cgaggtttta atggaaggtt tggagcctcg actggacgtt ggtaaatatc atcc 54
<210> 30
   <211> 46
   <212> DNA
   <213> Künstliche Sequenz
<220>
   <223> Mutante G12A
<400> 30
   cttgcaaaga atggcatcaa gctattccta accctcgttg ctggtg 46
<210> 31
   <211> 44
   <212> DNA
   <213> Künstliche Sequenz
<220>
   <223> Mutante H10A
<400> 31
   cgagggttag gaataccttg agcccattct ttgcaaggca acag 44
<210> 32
   <211> 54
   <212> DNA
   <213> Künstliche Sequenz
<220>
   <223> Mutante I45A
<400> 32
   ttggtaaata tcatccatag gtttagccag ttctgaacgt ggacgacatt gagg 54
<210> 33
   <211> 50
   <212> DNA
   <213> Künstliche Sequenz
<220>
   <223> Mutante K2A
<400> 33
   ccttgatgcc attctttgca aggcaacagt gccatatgta tatctccttc 50
<210> 34
   <211> 50
   <212> DNA
   <213> Künstliche Sequenz
<220>
   <223> Mutante K7A
<400> 34
   catatgaaac tgttgccttg cgcagaatgg catcaaggta ttcctaaccc 50
<210> 35
   <211> 40
   <212> DNA
   <213> Künstliche Sequenz
<220>
   <223> Mutante L24A
<400> 35
   gtattggtct tgtgcgcatt ccgcatcagc cccacaccag 40
<210> 36
   <211> 55
   <212> DNA
   <213> Künstliche Sequenz
<220>
   <223> Mutante M48A
<400> 36
   cgactggacg ttggtaaata tcatccgcag gtttaatcag ttctgaacgt ggacg 55
<210> 37
   <211> 43
   <212> DNA
   <213> Künstliche Sequenz
<220>
   <223> Mutante N60A
<400> 37
   cctcacgagg ttttaatgga agggctggaa actcgactgg acg 43
<210> 38
   <211> 45
   <212> DNA
   <213> Künstliche Sequenz
<220>
   <223> Mutante P65A
<400> 38
   gtggtgctcg agtgattcct cacgagcttt taatggaagg tttgg 45
<210> 39
   <211> 53
   <212> DNA
   <213> Künstliche Sequenz
<220>
   <223> Mutante R39A
<400> 39
   ggtttaatca gttctgaacg tggagcacat tgaggaatga aggcacagta ttg 53
<210> 40
   <211> 43
   <212> DNA
   <213> Künstliche Sequenz
<220>
   <223> Mutante Y31A
<400> 40
   gaggaatgaa ggcacaagct tggtcttgtg cgcattccag atc 43
<210> 41
   <211> 50
   <212> DNA
   <213> Künstliche Sequenz
<220>
   <223> Mutante C6A
<400> 41
   gggttaggaa taccttgatg ccattctttg gcaggcaaca gtttcatatg 50
<210> 42
   <211> 43
   <212> DNA
   <213> Künstliche Sequenz
<220>
   <223> Mutante C18A
<400> 42
   cattccagat cagccccaca ccaggcacga gggttaggaa tac 43
<210> 43
   <211> 38
   <212> DNA
   <213> Künstliche Sequenz
<220>
   <223> Mutante C20A
<400> 43
   cattccagat cagccccagc ccagcaacga gggttagg 38
<210> 44
   <211> 40
   <212> DNA
   <213> Künstliche Sequenz
<220>
   <223> Mutante C26A
<400> 44
   gtattggtct tgtgcggctt ccagatcagc cccacaccag 40
<210> 45
   <211> 44
   <212> DNA
   <213> Künstliche Sequenz
<220>
   <223> Mutante C32A
<400> 45
   gacattgagg aatgaaggca gcgtattggt cttgtgcgca ttcc 44
<210> 46
   <211> 46
   <212> DNA
   <213> Künstliche Sequenz
<220>
   <223> Mutante C38A
<400> 46
   cagttctgaa cgtggacgag cttgaggaat gaaggcacag tattgg 46
<210> 47
   <211> 16
   <212> PRT
   <213> Künstliche Sequenz
<220>
   <223> Peptid minimal 1
<400> 47
   Asp Asp Ile Tyr Gly Arg Pro Val Glu Phe Pro Asn Leu Pro Leu Lys
   1               5                   10                  15
<210> 48
   <211> 19
   <212> PRT
   <213> Künstliche Sequenz
<220>
   <223> Peptid B10
<400> 48
   Met Asp Asp Ile Tyr Gln Arg Pro Val Glu Phe Pro Asn Leu Pro Leu
   1 5 10 15
   Lys Pro Arg
<210> 49
   <211> 18
   <212> PRT
   <213> Künstliche Sequenz
<220>
   <223> Peptid B11
<400> 49
   Asp Asp Ile Tyr Gln Arg Pro Val Glu Phe Pro Asn Leu Pro Leu Lys
   1 5 10 15
   Pro Arg
<210> 50
   <211> 17
   <212> PRT
   <213> Künstliche Sequenz
<220>
   <223> Peptid B12
<400> 50
   Asp Ile Tyr Gln Arg Pro Val Glu Phe Pro Asn Leu Pro Leu Lys Pro
   1 5 10 15
   Arg
<210> 51
   <211> 16
   <212> PRT
   <213> Künstliche Sequenz
<220>
   <223> Peptid C1
<400> 51
   Ile Tyr Gln Arg Pro Val Glu Phe Pro Asn Leu Pro Leu Lys Pro Arg
   1 5 10 15
<210> 52
   <211> 15
   <212> PRT
   <213> Künstliche Sequenz
<220>
   <223> Peptid C2
<400> 52
   Tyr Gln Arg Pro Val Glu Phe Pro Asn Leu Pro Leu Lys Pro Arg
   1               5                   10                  15
<210> 53
   <211> 14
   <212> PRT
   <213> Künstliche Sequenz
<220>
   <223> Peptid C3
<400> 53
   Gln Arg Pro Val Glu Phe Pro Asn Leu Pro Leu Lys Pro Arg
   1               5                   10
<210> 54
   <211> 13
   <212> PRT
   <213> Künstliche Sequenz
<220>
   <223> Peptid C4
<400> 54
   Arg Pro Val Glu Phe Pro Asn Leu Pro Leu Lys Pro Arg
   1               5                   10
<210> 55
   <211> 12
   <212> PRT
   <213> Künstliche Sequenz
<220>
   <223> Peptid C5
<400> 55
   Pro Val Glu Phe Pro Asn Leu Pro Leu Lys Pro Arg
   1               5                   10
<210> 56
   <211> 11
   <212> PRT
   <213> Künstliche Sequenz
<220>
   <223> Peptid C6
<400> 56
   Val Glu Phe Pro Asn Leu Pro Leu Lys Pro Arg
   1               5                   10
<210> 57
   <211> 10
   <212> PRT
   <213> Künstliche Sequenz
<220>
   <223> Peptid C7
<400> 57
   Glu Phe Pro Asn Leu Pro Leu Lys Pro Arg
   1               5                   10
<210> 58
   <211> 19
   <212> PRT
   <213> Künstliche Sequenz
<220>
   <223> Peptid C8
<400> 58
   Pro Met Asp Asp Ile Tyr Gln Arg Pro Val Glu Phe Pro Asn Leu Pro
   1               5                   10                  15
   Leu Lys Pro
<210> 59
   <211> 18
   <212> PRT
   <213> Künstliche Sequenz
<220>
   <223> Peptid C9
<400> 59
   Pro Met Asp Asp Ile Tyr Gln Arg Pro Val Glu Phe Pro Asn Leu Pro
   1 5 10 15
   Leu Lys
<210> 60
   <211> 17
   <212> PRT
   <213> Künstliche Sequenz
<220>
   <223> Peptid C10
<400> 60
   Pro Met Asp Asp Ile Tyr Gln Arg Pro Val Glu Phe Pro Asn Leu Pro
   1 5 10 15
   Leu
<210> 61
   <211> 16
   <212> PRT
   <213> Künstliche Sequenz
<220>
   <223> Peptid C12
<400> 61
   Pro Met Asp Asp Ile Tyr Gln Arg Pro Val Glu Phe Pro Asn Leu Pro
   1 5 10 15
<210> 62
   <211> 15
   <212> PRT
   <213> Künstliche Sequenz
<220>
   <223> Peptid D1
<400> 62
   Pro Met Asp Asp Ile Tyr Gln Arg Pro Val Glu Phe Pro Asn Leu
   1 5 10 15
<210> 63
   <211> 14
   <212> PRT
   <213> Künstliche Sequenz
<220>
   <223> Peptid D2
<400> 63
   Pro Met Asp Asp Ile Tyr Gln Arg Pro Val Glu Phe Pro Asn
   1               5                   10
<210> 64
   <211> 13
   <212> PRT
   <213> Künstliche Sequenz
<220>
   <223> Peptid D3
<400> 64
   Pro Met Asp Asp Ile Tyr Gln Arg Pro Val Glu Phe Pro
   1               5                   10
<210> 65
   <211> 11
   <212> PRT
   <213> Künstliche Sequenz
<220>
   <223> Peptid D4
<400> 65
   Pro Met Asp Asp Ile Tyr Gln Arg Pro Val Glu
   1               5                   10
<210> 66
   <211> 10
   <212> PRT
   <213> Künstliche Sequenz
<220>
   <223> Peptid D5
<400> 66
   Pro Met Asp Asp Ile Tyr Gln Arg Pro Val
   1               5                   10
<210> 67
   <211> 20
   <212> PRT
   <213> Künstliche Sequenz
<220>
   <223> Peptid A2
<400> 67
   Pro Met Asp Asp Ile Tyr Gln Arg Pro Val Glu Phe Pro Asn Leu Pro
   1               5                   10                  15
   Leu Lys Pro Ala
               20
<210> 68
   <211> 20
   <212> PRT
   <213> Künstliche Sequenz
<220>
   <223> Peptid A3
<400> 68
   Pro Met Asp Asp Ile Tyr Gln Arg Pro Val Glu Phe Pro Asn Leu Pro
   1               5                   10                  15
   Leu Lys Ala Arg
               20
<210> 69
   <211> 20
   <212> PRT
   <213> Künstliche Sequenz
<220>
   <223> Peptid A4
<400> 69
   Pro Met Asp Asp Ile Tyr Gln Arg Pro Val Glu Phe Pro Asn Leu Pro
   1               5                   10                  15
   Leu Ala Pro Arg
               20
<210> 70
   <211> 20
   <212> PRT
   <213> Künstliche Sequenz
<220>
   <223> Peptid A5
<400> 70
   Pro Met Asp Asp Ile Tyr Gln Arg Pro Val Glu Phe Pro Asn Leu Pro
   1 5 10 15
   Ala Lys Pro Arg
   20
<210> 71
   <211> 20
   <212> PRT
   <213> Künstliche Sequenz
<220>
   <223> Peptid A6
<400> 71
   Pro Met Asp Asp Ile Tyr Gln Arg Pro Val Glu Phe Pro Asn Leu Ala
   1 5 10 15
   Leu Lys Pro Arg
   20
<210> 72
   <211> 20
   <212> PRT
   <213> Künstliche Sequenz
<220>
   <223> Peptid A7
<400> 72
   Pro Met Asp Asp Ile Tyr Gln Arg Pro Val Glu Phe Pro Asn Ala Pro
   1 5 10 15
   Leu Lys Pro Arg
   20
<210> 73
   <211> 20
   <212> PRT
   <213> Künstliche Sequenz
<220>
   <223> Peptid A8
<400> 73
   Pro Met Asp Asp Ile Tyr Gln Arg Pro Val Glu Phe Pro Ala Leu Pro
   1               5                   10                  15
   Leu Lys Pro Arg
               20
<210> 74
   <211> 20
   <212> PRT
   <213> Künstliche Sequenz
<220>
   <223> Peptid A9
<400> 74
   Pro Met Asp Asp Ile Tyr Gln Arg Pro Val Glu Phe Ala Asn Leu Pro
   1               5                   10                  15
   Leu Lys Pro Arg
               20
<210> 75
   <211> 20
   <212> PRT
   <213> Künstliche Sequenz
<220>
   <223> Peptid A10
<400> 75
   Pro Met Asp Asp Ile Tyr Gln Arg Pro Val Glu Ala Pro Asn Leu Pro
   1               5                   10                  15
   Leu Lys Pro Arg
               20
<210> 76
   <211> 20
   <212> PRT
   <213> Künstliche Sequenz
<220>
   <223> Peptid A11
<400> 76
   Pro Met Asp Asp Ile Tyr Gln Arg Pro Val Ala Phe Pro Asn Leu Pro
   1 5 10 15
   Leu Lys Pro Arg
   20
<210> 77
   <211> 20
   <212> PRT
   <213> Künstliche Sequenz
<220>
   <223> Peptid A12
<400> 77
   Pro Met Asp Asp Ile Tyr Gln Arg Pro Ala Glu Phe Pro Asn Leu Pro
   1 5 10 15
   Leu Lys Pro Arg
   20
<210> 78
   <211> 20
   <212> PRT
   <213> Künstliche Sequenz
<220>
   <223> Peptid B1
<400> 78
   Pro Met Asp Asp Ile Tyr Gln Arg Ala Val Glu Phe Pro Asn Leu Pro
   1 5 10 15
   Leu Lys Pro Arg
   20
<210> 79
   <211> 20
   <212> PRT
   <213> Künstliche Sequenz
<220>
   <223> Peptid B2
<400> 79
   Pro Met Asp Asp Ile Tyr Gln Ala Pro Val Glu Phe Pro Asn Leu Pro
   1 5 10 15
   Leu Lys Pro Arg
   20
<210> 80
   <211> 20
   <212> PRT
   <213> Künstliche Sequenz
<220>
   <223> Peptid B3
<400> 80
   Pro Met Asp Asp Ile Tyr Ala Arg Pro Val Glu Phe Pro Asn Leu Pro
   1 5 10 15
   Leu Lys Pro Arg
   20
<210> 81
   <211> 20
   <212> PRT
   <213> Künstliche Sequenz
<220>
   <223> Peptid B4
<400> 81
   Pro Met Asp Asp Ile Ala Gln Arg Pro Val Glu Phe Pro Asn Leu Pro
   1 5 10 15
   Leu Lys Pro Arg
   20
<210> 82
   <211> 20
   <212> PRT
   <213> Künstliche Sequenz
<220>
   <223> Peptid B5
<400> 82
   Pro Met Asp Asp Ala Tyr Gln Arg Pro Val Glu Phe Pro Asn Leu Pro
   1 5 10 15
   Leu Lys Pro Arg
   20
<210> 83
   <211> 20
   <212> PRT
   <213> Künstliche Sequenz
<220>
   <223> Peptid B6
<400> 83
   Pro Met Asp Ala Ile Tyr Gln Arg Pro Val Glu Phe Pro Asn Leu Pro
   1 5 10 15
   Leu Lys Pro Arg
   20
<210> 84
   <211> 20
   <212> PRT
   <213> Künstliche Sequenz
<220>
   <223> Peptid B7
<400> 84
   Pro Met Ala Asp Ile Tyr Gln Arg Pro Val Glu Phe Pro Asn Leu Pro
   1 5 10 15
   Leu Lys Pro Arg
   20
<210> 85
   <211> 20
   <212> PRT
   <213> Künstliche Sequenz
<220>
   <223> Peptid B8
<400> 85
   Pro Ala Asp Asp Ile Tyr Gln Arg Pro Val Glu Phe Pro Asn Leu Pro
   1               5                   10                  15
   Leu Lys Pro Arg
               20
<210> 86
   <211> 20
   <212> PRT
   <213> Künstliche Sequenz
<220>
   <223> Peptid B9
<400> 86
   Ala Met Asp Asp Ile Tyr Gln Arg Pro Val Glu Phe Pro Asn Leu Pro
   1               5                   10                  15
   Leu Lys Pro Arg
               20
<210> 87
   <211> 16
   <212> PRT
   <213> Künstliche Sequenz
<220>
   <223> Peptid 67
<400> 87
   Met Asp Asp Ile Tyr Gln Arg Pro Val Glu Phe Pro Asn Leu Pro Leu
   1               5                   10                  15
<210> 88
   <211> 14
   <212> PRT
   <213> Künstliche Sequenz
<220>
   <223> Peptid 68
<400> 88
   Asp Asp Ile Tyr Gln Arg Pro Val Glu Phe Pro Asn Leu Pro
   1               5                   10
<210> 89
   <211> 15
   <212> PRT
   <213> Künstliche Sequenz
<220>
   <223> Peptid 69
<220>
   <221> MISC_FEATURE
   <222> (7)..(7)
   <223> Xaa ist jede beliebige Aminosäure
<220>
   <221> MISC_FEATURE
   <222> (9)..(9)
   <223> Xaa ist jede beliebige Aminosäure
<220>
   <221> MISC_FEATURE
   <222> (12)..(12)
   <223> Xaa ist jede beliebige Aminosäure
<400> 89
   Asp Asp Ile Tyr Gln Arg Xaa Val Xaa Phe Pro Xaa Leu Pro Leu
   1               5                   10                  15
<210> 90
   <211> 486
   <212> DNA
   <213> Künstliche Sequenz
<220>
   <223> Tridegin DNA für Pichia Expression
<400> 90
   atgagatttc cttcaatttt tactgctgtt ttattcgcag catcctccgc attagctgct     60
   ccagtcaaca ctacaacaga agatgaaacg gcacaaattc cggctgaagc tgtcatcggt    120
   tactcagatt tagaagggga tttcgatgtt gctgttttgc cattttccaa cagcacaaat    180
   aacgggttat tgtttataaa tactactatt gccagcattg ctgctaaaga agaaggggta    240
   tctctcgaga aaagaaaact gttgccttgc aaagaatggc atcaaggtat tcctaaccct    300
   cgttgctggt gtggggctga tctggaatgc gcacaagacc aatactgtgc cttcattcct    360
   caatgtcgtc cacgttcaga actgattaaa cctatggatg atatttacca acgtccagtc    420
   gagtttccaa accttccatt aaaacctcgt gaggaatcac tcgaacacca ccaccaccac    480
   cactga                                                               486
<210> 91
   <211> 486
   <212> DNA
   <213> Künstliche Sequenz
<220>
   <223> Tridegin DNA für Pichia Expression 2
<400> 91
   atgagatttc cttcaatttt tactgctgtt ttattcgcag catcctccgc attagctgct     60
   ccagtcaaca ctacaacaga agatgaaacg gcacaaattc cggctgaagc tgtcatcggt    120
   tactcagatt tagaagggga tttcgatgtt gctgttttgc cattttccaa cagcacaaat    180
   aacgggttat tgtttataaa tactactatt gccagcattg ctgctaaaga agaaggggta    240
   tctctcgaga aaagaaaact gttgccttgc aaagaatggc atcaaggtat tcctaaccct    300
   cgttgctggt gtggggctga tctggaatgc gcacaagacc aatactgtgc cttcattcct    360
   caatgtcgtc cacgttcaga actgattaaa cctatggatg atatttacca acgtccagtc    420
   gagtttccaa accttccatt aaaacctctg gaggaatcac tcgaacacca ccaccaccac    480
   cactga                                                               486
<210> 92
   <211> 15
   <212> PRT
   <213> Künstliche Sequenz
<220>
   <223> Peptid minimal 2
<400> 92
   Asp Asp Ile Tyr Gly Arg Pro Val Glu Phe Pro Asn Leu Pro Leu
   1               5                   10                  15

## Beschreibung

Die vorliegende Erfindung betrifft modifizierte Tridegine, Polypetide abgeleitet von SEQ ID No.1, wobei die Modifikation darin besteht, dass der N- und/oder C-Terminus deletiert sind, wobei das verbleibende Polypeptid mindestens die Aminosäuresequenz DDIYQRXVXFPXLPL (SEQ ID NO. 89) enthält und gegebenenfalls mindestens ein Cysteinrest und/oder eine der folgenden Aminosäuren - Lys2, Lys7, His10, Gly12, Leu24, Tyr31, Phe34, Arg39, Ile45, Met48, Pro55, Asn60, Pro65, Arg66 - durch eine andere Aminosäure substituiert ist und/oder eine kovalente Verknüpfung mit Polyethylenglykol besteht. Die erfindungsgemäßen Polypeptide sind neue Inhibitoren von Transglutaminasen, insbesondere des Faktors XIIIa, des terminalen Enzyms der BlutgerinnungsKaskade. Die vorliegende Erfindung betrifft ebenso Verfahren zur Herstellung dieser Inhibitoren, sowie deren Verwendung als Transglutaminase-Inhibitoren.

Transglutaminasen (EC 2.3.2.13) katalysieren die Bildung von Amidbindungen innerhalb einer oder zwischen verschiedenen Polypeptidketten nach dem folgenden Reaktionsschema:

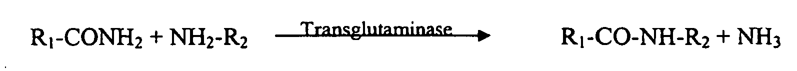

Sie katalysieren also die Quervernetzung von Proteinen, durch Bildung von γ-Glutamyl-ε-Lysin Bindungen zwischen zwei Polypeptidketten, und tragen damit zur Stabilisierung von vielen Proteinaggregaten bei.

Eine Transglutaminase mit großer klinischer Bedeutung ist der Faktor XIIIa. Faktor XIIIa ist das terminale Enzym der Blutgerinnungskaskade und vernetzt Fibrinpolymere eines "weichen" Blutthrombus durch Transglutaminierung kovalent. Faktor XIIIa sorgt außerdem für die kovalente Bindung von α₂-Antiplasmin durch Transglutaminierung an das Fibrinnetzwerk. Ein derartig quervernetzter und modifizierter Blutthrombus wird als "harter" Blutthrombus bezeichnet und kann von fibrinolytischen Enzymen nicht so schnell aufgelöst werden wie ein "weicher" Blutthrombus aus Fibrinpolymeren ohne kovalente Quervernetzung. Damit trägt der Faktor XIIIa entscheidend zur Stabilisierung eines Blutthrombus bei.

Inhibitoren des Faktors XIIIa verhindern die Quervernetzungsreaktion zwischen den unterschiedlichen Ketten des Fibrinnetzwerkes und auch die kovalente Bindung von α₂-Antiplasmin und erleichtern damit sowohl die präventive Behandlung von thrombotischen Ereignissen, als auch die thrombolytische Behandlung.

Mehrere Inhibitoren von Transglutaminasen sind bereits im Stand der Technik beschrieben (eine Auswahl findet sich in Tabelle 1). Es handelt sich hierbei um
- Immunglobuline, die an Transglutaminasen binden,
- niedermolekulare chemische Verbindungen, die mit Cysteinen reagieren,
- niedermolekulare Amine, die mit natürlichen Substraten kompetitieren und
- aktive Fraktionen, gewonnen aus Blutegeln der Spezies *Haementeria ghilianii*.

**Tabelle 1: Auswahl publizierter und patentierter Inhibitoren des Faktors XIIIa**

| **Inhibitor** | Affinität (IC₅₀) | Referenz |
|---|---|---|
| Cerulenin | 29 µM | US 5,710,174 |
| ZG-1400 | 5,7 µM | US 5,710,174 |
| Imidazol Verbindungen | > 80 nM | US 4,968,713 |
| 2-(1-Acetonylthio)-5-Methylthiazolo [2,3-b]1,3,4-Thiadiazolium Perchlorat (L-722,151) | unbekannt | |
| Monodansyl Cadaverin | unbekannt | US 5,124,358 |
| Isothiocyanate | | WO 9213530 |
| Aktive Fraktion (Tridegin?) | 3.4 nM | US 6,025,330 |

Immunglobuline, die gegen den Faktor XIII gerichtet sind, wurden z.B. in US 5,470,957 offenbart. Dort wurden monoklonale Antikörper gegen eine Untereinheit des Faktors XIIIa hergestellt und beobachtet, daß derartige Antikörper die Aktivierung von Faktor XIII durch Thrombin inhibierten. Allerdings sind für einen therapeuthischen Einsatz derartiger Antikörper umfangreiche Veränderungen, wie z.B. die Herstellung von humanen Chimären, normalerweise notwendig.

Eine weitere Klasse von Inhibitoren besteht aus niedermolekularen, reaktiven chemischen Verbindungen, die irreversibel an das aktive Zentrum des Faktors XIIIa, einen Cysteinrest, binden. Derartige Verbindungen, offenbart in WO 92/13530, haben allerdings den Nachteil, dass sie sehr reaktiv und *in vivo* relativ instabil sind. Sie reagieren auch mit Cysteinresten anderer Proteine und sind damit nicht spezifisch für den Faktor XIIIa. Sie finden somit als pharmazeutische Wirkstoffe keine Anwendung.

Weitere Transglutaminase-Inhibitoren sind niedermolekulare Amine, wie in WO 91/10427 offenbart, die als kompetitive Substrate der Transglutaminasereaktion wirken. Sie werden allerdings durch die Transglutaminierungsreaktion verbraucht und verändern die Funktionalität der Proteine, an die sie durch die Transglutaminasereaktion gekoppelt werden, auf nicht vorhersehbare Weise. Außerdem müssen sie in einer relativ hohen Konzentration von etwa 200 µM eingesetzt werden, was ihren therapeutischen Wert beschränkt.

Aus den Speicheldrüsen von Blutegeln der Spezies *Haementeria ghilianii* wurde eine Fraktion isoliert, die den Faktor XIIIa mit hoher Affinität und Spezifität hemmt (offenbart in US 6,025,330). In den aufgereinigten, aktiven Fraktionen befanden sich mindestens zwei verschiedene Proteine, wobei ein Protein mit einer ungefähren Größe von 7-8 kDa den Hauptproteinbestandteil der aktiven Fraktionen darstellte. Die Primärsequenz dieses 66 Aminosäuren langen Polypeptides wurde durch proteinbiochemische Methoden annährend bestimmt. Dieses Polypeptid wurde Tridegin genannt und es wurde angenommen, dass dieses Polypeptid Transgluaminasen im allgemeinen und insbesondere den Faktor XIIIa inhibiert.

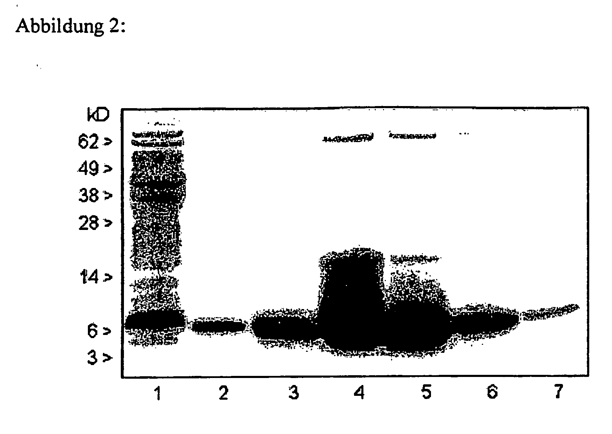

Es wurde allerdings kein Versuch unternommen, das Tridegin von den anderen, noch in der aktiven Fraktion anwesenden, Proteinen abzutrennen, so dass nicht ausgeschlossen ist, dass es diese Proteine sind, die den Faktor XIIIa inhibieren (siehe insbesondere Finney et al., (Finney et al., Biochem. Journal 324, 797-805 (1997), Figur 2, Spur 3)). Es wurde nicht einmal ermittelt, ob die den Faktor XIIIa inhibierende Aktivität peptidischer Natur ist. Folglich könnten andere, in den aktiven Fraktionen vorliegende, Biopolymere die eigentliche, den Faktor XIIIa inhibierende, Aktivität darstellen, sofern sie bei der Analyse auf einem SDS-Gel nicht auffallen, wie komplexe Zucker oder Lipide oder Glykolipide. Somit wurde in diesen Publikationen nicht gezeigt, dass Tridegin den Faktor XIIIa inhibiert.

Folglich ist es völlig unklar, ob ein rekombinant hergestellte, z.B. im Prokaryonten *Escherichia coli* exprimiertes Tridegin als Inhibitor für den Faktor XIIIa funktionell wäre. Sowohl in US 6,025,330 (4, 12-18) als auch im entsprechenden wissenschaftlichen Artikel (Finney et al., Biochem. Journal 324, 797-805 (1997)), wurde beobachtet, daß das aus Blutegeln der Spezies *Haementeria ghilianii* aufgereinigte Tridegin post-translational modifiziert war (Finney et al., Biochem. Journal (1997) 324, 800, rechte Spalte, letzter Satz). Obwohl bekannt ist, daß derartige Modifikationen gerade bei sekretierten Proteinen, zu denen auch das Tridegin zählt, für die Funktion oft notwendig sind ( z.B. beschrieben in Kemball-Cook et al., Gene, 139(2): 275-279 (1994) oder Pang et al., Endocrinology 140(11): 5102-5111 (1999)), wurde in diesen beiden Publikationen nicht darauf eingegangen, inwieweit posttranslationale Modifikationen für die angenommene Funktion von Tridegin nötig sind. Derartige Modifikationen fehlen aber in Proteine, die rekombinant in *Escherichia coli* hergestellt wurden. Darum sind gerade Proteine, die normalerweise extrazellulär lokalisiert sind, oft inaktiv, wenn sie in einem heterologen System exprimiert werden.

WO 96/34890 beschreibt Inhibitoren, die aus Geweben oder Schnitten von Blutegeln erhältlich sind und die die folgende N-terminale Sequenz aufweisen: NH2-Lys-Leu-Leu-Pro-Cys-Lys-Glu-Y-His-Gln-Gly-Ile-Pro-Asn-Pro-Arg-, wobei Y jede beliebige Aminosäure darstellt. Auch beschrieben sind pharmazeutisch verträgliche Salze, Derivate, Analoge, Homologe und biologische Vorläufer der genannten Sequenz sowie ihre mögliche Verwendung zur Behandlung von Morbus Crohn, Tumoreinnistung, Arteriosklerose, thrombotischer Mikroangiopathie, fibrösem Wachstum der Haut, Akne, Narbenbildung, membranärer Glomerulonephritis, Katarakten oder Infektionen mit Nematoden sowie insbesondere zur Reduktion der Stabilität von Thromben, so dass sie für thrombolytische Mittel empfänglicher sind.

WO 00/49039 beschreibt eine neue Technik zur Aufreinigung von Fusionsproteinen und offenbart dabei eine synthetische DNA-Sequenz, die für Tridegin kodiert. Außerdem wurde Tridegin als Fusionsprotein mit Glukosedehydrogenase exprimiert und aufgereinigt, die Aktivität des Fusionsproteins als Inhibitor des Faktor XIIIa wurde allerdings nicht getestet.

Die Erfindung hat daher die Aufgabe, neue Polypeptid-Inhibitoren von Transglutaminasen rekombinant oder synthetisch in ausreichenden Mengen und in reiner Form bereitzustellen.

Überraschenderweise stellte sich das in einem heterologen Systen, z.B. in *Escherichia coli*, exprimierte und aufgereinigte Tridegin Polypeptid als effektiver Transglutaminaseinhibitor, insbesondere als Inhibitor von Faktor XIIIa, vor allem von humanem Faktor XIIIa, heraus. Damit wurde erstmals gezeigt, dass das Tridegin Polypeptid tatsächlich ein Transglutaminaseinhibitor, insbesondere ein Inhibitor von Faktor XIIIa, vor allem von humanem Faktor XIIIa, ist. Überraschenderweise zeigten auch Tridegin Polypeptide mit einer oder mehreren Modifikationen eine Aktivität als Transglutaminaseinhibitor, insbesondere als Inhibitor von Faktor XIIIa, vor allem von humanem Faktor XIIIa. Überraschenderweise zeigte sich, dass das in der Hefe *Pichia pastoris* exprimierte rekombinante Tridegin Polypeptid ein noch effektiverer Transglutaminase-Inhibitor ist, als das zuvor erwähnte rekombinante Tridegin Polypeptid aus *Escherichia coli.*

Gegenstand der vorliegenden Erfindung ist daher ein modifiziertes Tridegin Polypeptid abgeleitet von SEQ ID No.1 mit einer oder mehreren Modifikationen, wie in den Ansprüchen definiert.

Unter einem Polypeptid versteht man im Sinne dieser Erfindung Peptide mit mehr als 15 Aminosäuren (AS) und weniger als 2000 Aminosäuren, bevorzugt Peptide mit mehr als 15 AS und weniger als 500 AS, insbesondere Peptide mit mehr als 16, 17, 18, 19 oder 20 AS und weniger als 400, 300, 200, 100, 80, 60, 50, 40 oder 30 AS.

Unter einer Modifikation des Polypeptides gemäß SEQ ID NO: 1 versteht man im Sinne dieser Erfindung eine Veränderung des wildtyp Tridegin Polypeptides durch eine Deletion des N- und/oder C-Terminus und gegebenenfalls durch die Substitution mindestens eines Cysteinrestes durch eine andere Aminosäure und/oder die Substitution mindestens einer der folgenden Aminosäuren - Lys2, Lys7, His10, Gly12, Leu24, Typ31, Phe34, Arg39, Ile45, Met48, Pro55, Asn60, Pro65, Arg66 - durch eine andere Aminosäure und/oder eine kovalente Verknüpfung mit Polyethylenglykol.

Unter einer Substitution versteht man im Sinne dieser Erfindung das Ersetzen einer Aminosäure an einer bestimmten Stelle der Aminosäuresequenz eines Polypeptides durch eine andere Aminosäure, vorzugsweise durch eine der anderen 19 natürlichen Aminosäuren.

Unter einer Deletion versteht man im Sinne dieser Erfindung das Entfernen von N- und/oder C-terminalen Bereichen der Aminosäuresequenz des Tridegins Polypeptides, z.B. das Entfernen von insgesamt mehr als 5, 10, 15, 20, 25, 30, 35 oder sogar 40 Aminosäuren (wobei hier die Summe der am N- und C-Terminus entfernten Aminosäuren gemeint ist), wobei das verbleibende Polypeptid noch mindestens die Aminosäuresequenz DDIYGRPVEFPNLPL (SEQ ID No. 92) oder DDIYGRPVEFPNLPLK (SEQ ID No. 47) enthält.

Überraschenderweise zeigten die modifizierten Tridegin Polypeptide im Vergleich zum in *Escherichia coli* exprimierten wildtyp Tridegin Polypeptid folgende vorteilhafte Eigenschaften.

Während das aus *Escherichia coli* gewonnene, rekombinante wildtyp Tridegin Polypeptid auch hochmolekulare Aggregate bildete, zeigten diejenigen modifizierten Tridegin Polypeptide, bei denen mindestens ein Cysteinrest, bevorzugt ein bis vier Cysteinreste, besonders bevorzugt drei oder vier Cysteinreste durch eine andere Aminosäure, vorzugsweise eine kleine Aminosäure wie Valin, Alanin, Glycin oder Serin, besonders bevorzugt durch Alanin oder Serin, vor allem durch Alanin, substituiert war, eine verminderte Aggregatbildung. Dies wurde in vergleichenden, analytischen Gelfiltrationsläufen festgestellt. Für die experimentellen Bedingungen der Gelfiltrationsläufe wird auf das Ausführungsbeispiel 4 verwiesen.

Außerdem zeigen besagte modifizierte Tridegin Polypeptide bei der Lagerung bei 4°C eine langsamere Bildung der hochmolekularen Aggregate als das wildtyp Tridegin. Dies wird in vergleichenden, analytischen Gelfiltrationsläufen festgestellt. Für die experimentellen Bedingungen der Gelfiltrationsläufe wird auf das Ausführungsbeispiel 4 verwiesen.

Während das in *Pichia pastoris* exprimierte wildtyp Tridegin-Polypeptid am extremen C-Terminus von einer oder mehreren Proteasen gespalten wurde, konnte die Variante Arg66Leu überraschenderweise vollständig exprimiert und gereinigt werden. Im Vergleich zum in *Pichia pastoris* exprimierte wildtyp Tridegin Polypeptid wurde eine im wesentliche unveränderte inhibitorische Aktivität beobachtet, die jedoch besser war, als die des in *E.coli* exprimierten wildtyp Tridegin-Polypeptids.

Tridegin Polypeptide, die von ihrem Wirt sekretiert wurden, können eine höhere spezifische Aktivität besitzen als intrazellulär produzierte Tridegin Polypeptide, wie hier am Beispiel des aus *Pichia Pastoris* gewonnenen Tridegin gezeigt wird, und können somit besonders vorteilhaft sein. Darum sind rekombinante Tridegin Polypeptide, die durch Sekretion von einem Wirt erhältlich sind, ein Gegenstand der Erfindung, insbesondere, wenn sie durch Sekretion von einem Wirt erhalten sind. Ebenso ist ein Verfahren zur Herstellung von Tridegin Polypeptiden, das von Tridegin Polypeptiden ausgeht, die in einem Sekretionsschritt von ihrem Wirt an die Zellaussenseite, insbesondere an das Medium abgegeben wurden, ein Gegenstand der Erfindung. Das kann alle gentechnischen Verfahren zur Herstellung von Tridegin Polypeptid, bei denen ein Sekretionsschritt des Tridegin Polypeptides umfasst ist, betreffen. Ein solcher Sekretionsschritt kann vorliegen, wenn das Tridegin Polypeptid im Wirt eine Zellmembran überquert. Der Sekretionsschritt kann während der Synthese des Tridegin Polypeptides erfolgen oder auch nachdem das Polypeptid bereits in der Zelle vorliegt.

Sekretion eines rekombinanten Polypeptids der Erfindung, exprimiert in einem gentechnisch manipulierbaren Wirt, kann man erreichen, indem man mit geeigneten molekularbiologischen Methoden, z.B. beschrieben in Sambrook et al., "Molecular Cloning: A Laboratory Manual." Third edition (2001) CSHL Press, einen DNA Expressionsvektor erzeugt, der eine Nukleinsäure umfasst, die unter der Kontrolle eines für die Expression im entsprechenden Wirt geeigneten Promotor steht und die für ein Polypeptid kodiert, das, bevorzugt an seinem N-terminus, ein sogenanntes Signalpeptid umfasst. Signalpeptide können von der zellulären Sekretionsmaschinerie erkannt werden und können die Translokation eines Proteins durch eine Zellmembran vermitteln. Der Translokationsvorgang wird im allgemeinen durch die Translokationsmaschinerie vermittelt, die eine Art Kanal für bestimmte Proteine durch die Lipidmembran bildet. Bei der Translokation durch diesen Kanal wird das Signalpeptid eines sekretierten Proteins im allgemeinen, jedoch nicht in allen Fällen, abgetrennt. Die Funktionsweise und die Bestandteile der Translokationsmaschinerie werden in Rapoport TA., et al., Annu. Rev. Biochem. (1996) 65: 271-303 diskutiert, ebenso Gemeinsamkeiten und Unterschiede der Translokationsmaschinerie bei Eukaryonten und Prokaryonten.

Der Wirt für die Expression und Sekretion eines Polypeptides der Erfindung kann ein jeder mikrobiologischer Wirt sein, aber auch höhere eukaryontische Zellen in Kultur, wie z.B. humane Zellen (z.B. HeLa Zellen) oder Insektenzellen (z.B. Insektenzellen, die mit Bakulovirus infiziert werden können, zur ektopischen Proteinexpression), solange der Wirt mit gentechnischen Methoden manipulierbar ist und in der Lage ist, rekombinante Proteine zu sekretieren. Der mikrobielle Wirt kann ein Archäbakterium, ein Eubakterium oder ein niederer Eukaryont wie z.B. ein Pilz (wie Acrasiomyceten, Myxomyceten, Phycomyceten, Ascomyceten, Basidomyceten oder Fungi imperfecti, insbesondere Hefen wie *Pichia Pastoris* oder *Saccharomyces cerevisiae*) oder ein Protist (wie Flagellaten, Rhizopoden, Sporozoen oder Ciliaten, insbesondere auch Schleimpilze wie *Dictostelium discoideum*) sein. Auch Zellen höherer Eukaryonten, wie z.B. Säuger-Zelllinien, können durch Transfektion mit geeigneten Vektoren Proteine im Cytoplasma (z.B. pcDNA3.1, Invitrogen Inc.) oder auch sekretorisch (z.B. pSecTag2, Invitrogen Inc.) exprimieren (siehe z.B. "Mammalian Cell Biotechnology: A Practical Approach von M. Butler (Herausgeber), IRL Press, Oxford-New York-Tokyo, Seite 9, Zeile 23: Beispiele 6 und 7). Als Wirtszellen kommen dafür u.a. CHO-Zellen oder HEK293-Zellen in Betracht.

Insbesondere kann der Wirt ein gram-negatives Bakterium, wie z.B. *Escherichia coli* oder *Serratia marcescens*, sein. Bei diesen Bakterien können sekretierte, rekombinante Protein an das Periplasma abgegeben werden und die Gewinnung dieser sekretierten Proteine ist möglich ohne die Wirtszelle selbst aufzuschliessen. Geeignete Signalpeptide für die Verwendung in gram-negativen Bakterien sind, z.B. für *Escherichia coli,* in Pines O. und Inouye M., Mol. Biotechnol. (1999) 12: 25-34 beschrieben.

Insbesondere kann der Wirt ein gram-positives Bakterium, wie z.B. *Bacillus subtilis* und verwandte Bacillus species, wie *B. amyloliquefaciens* oder *B. licheniformis*, sein, da diese Bakterien ebenfalls Proteine an das Kulturmedium abgeben können. Geeignete Signalpeptide für die Verwendung in gram-positiven Bakterien sind z.B. für *B. subtilis* in Tjalsma H., et al., Micobiology and Molecular Biology Reviews, (2000) 64: 515-547 beschrieben.

Bevorzugt ist auch ein niederer Eukaryont als Wirt, da von diesen sekretierte, rekombinante Proteine an das Medium abgegeben werden können und somit ebenso der Aufschluss der Wirtszelle nicht immer nötig ist. Geeignete Signalpeptide für die Verwendung in Eukaryonten sind z.B. beschrieben in Rapoport TA. et al., Annu. Rev. Biochem. (1996) 65: 271-303. Ausserdem sei auf das im Beispiel 6 verwendete Signalpeptid des Alpha Faktors, sowie auf Kjeldsen T., Appl. Microbiol. Biotechnol. (2000) 54(3): 277-86 und Brake A.J. Biotechnology (1989) 13:269-80 hingewiesen.

Ohne an eine bestimmte Theorie gebunden zu sein, scheint das sekretierte Tridegin Polypeptid durch den Durchtritt des Polypeptides durch die Translokationsmaschinerie, die evolutionär zwischen Bakterien und Eukaryonten in Teilen konserviert ist, eine Faltung zu erhalten, die vorteilhaft ist. Ausserdem könnte es sein, dass Qualitätskontrollmechanismen, die bei der Sekretion wirksam sind, dafür sorgen, dass das sekretierte Tridegin Polypeptid im Wesentlichen frei von falsch gefaltetem Tridegin Polypeptid ist, wodurch eine hohe spezifische inhibitorische Aktivität der sekretierten Tridegin Polypeptide erreicht werden kann. Ausserdem gelangt das Tridegin-Polypeptid duch den Sekretionsschritt aus dem reduzierenden Millieu des Cytoplasmas in oxidierende Zell-Kompartimente, was die Ausbildung von Disulfid-Brücken erleichtert.

Diejenigen modifizierten Tridegin Polypeptide, bei denen mindestens eine, bevorzugt eine bis zehn, besonders bevorzugt eine bis sechs, vorallem eine bis drei, insbesondere aber nur eine der folgenden Aminosäuren - Lys2, Lys7, His10, Gly12, Leu24, Tyr31, Phe34, Arg39, Ile45, Met48, Pro55, Asn60, Pro65, Arg66 - durch eine andere Aminosäure, vorzugsweise eine kleine Aminosäure wie Valin, Alanin, Glycin oder Serin, besonders bevorzugt durch Alanin und Glycin, vor allem durch Alanin substituiert wurden, zeigen überraschenderweise eine im Vergleich zum wildtyp Tridegin verminderte Antigenizität, überraschenderweise bei einer mit dem wildtyp Tridegin Polypeptid vergleichbaren inhibitorischen Aktivität auf den humanen Faktor XIIIa, was wiederum überraschend war.

Bei der Suche nach einer vom wildtyp Tridegin Polypeptid abgeleiteten minimalen, den Faktor XIIIa inhibierenden Aminosäuresequenz, ergab sich überraschenderweise, dass Polypeptide, die mindestens die Aminosäuresequenz DDIYQRXVXFPXLPL, insbesondere die Aminosäuresequenz DDIYQRPVEFPNLPL oder DDIYGRPVEFPNLPLK, enthielten, eine inhibitorische Wirkung auf den humanen Faktor XIIIa aufwiesen. So inhibierten selbst Polypeptide von nur 16 Aminosäuren Länge, welche die oben genannte Aminosäuresequenz enthielten, den humanen Faktor XIIIa. Varianten der vom wildtyp Tridegin-Polypetid abgeleiteten inhibitorisch aktiven Polypeptide, bei der jeweils eine Aminosäure durch Alanin ersetzt wurde, bestätigten diese Ergebnisse. Zudem ermöglichen diese Experimente die Ableitung der für die inhibitorischen Wirkung essentiellen Reste.

Die Auswirkungen des Alanin-Austauschs im Ursprungspolypeptid, SEQ ID No. 25 (Ausgetauschte Reste in der Sequenz mit X markiert) können so zusammengefasst werden:
Ausgangssequenz (SEQ ID No. 25): PMDDIYQRPVEFPNLPLKPR
Austausch ohne Aktivitätsverminderung für folgende Aminosäuren X: XXDDIYQRXVXFPXLPLKXX
Austausch mit geringer Aktivitätsverminderung für folgende Aminosäuren X: PMXXIYXXPXEXXNXXLXPR
Austausch mit stärkerer Aktivitätsverminderung für folgende Aminosäuren X: PMDDXXQRPVEFPNLPXKPR

Aus diesen Ergebnissen folgt, dass das minimale, FXIIIa inhibierende Polypeptid folgende Sequenz hat: DDIYQRXVXFPXLPL (SEQ ID No. 89), wobei die mit X bezeichneten Aminosäuren unabhängig voneinander beliebige Aminosäuren, vorzugsweise ausgewählt aus den natürlichen Aminosäuren, insbesondere kleine Aminosäuren wie Valin, Alanin, Glycin, oder Serin, besonders bevorzugt Alanin und Glycin, vor allem aber Alanin sein können. Ein, zwei oder drei der mit X bezeichneten Aminosäuren in der obigen Sequenz mit der SEQ ID NO. 89 können auch die wildtyp-Aminosäuren für die entsprechende Stelle sein.

Kurze Polypeptide, mit weniger als 40, bevorzugt mit weniger als 30, besonders bevorzugt mit weniger als 25 Aminosäuren, die mindestens die Aminosäuresequenz DDIYQRXVXFPXLPL (SEQ ID No. 89), wobei die mit X bezeichneten Aminosäuren unabhängig voneinander beliebige Aminosäuren, vorzugsweise ausgewählt aus den natürlichen Aminosäuren, insbesondere kleine Aminosäuren wie Valin, Alanin, Glycin, oder Serin, besonders bevorzugt Alanin und Glycin, vor allem aber Alanin sein können und ein, zwei oder drei der mit X bezeichneten Aminosäuren in der obigen Sequenz mit der SEQ ID NO. 89 auch die wildtyp-Aminosäuren für die entsprechende Stelle sein können, insbesondere solche, die die Aminosäuresequenz DDIYQRPVEFPNLPL oder DDIYQRPVEFPNLPLK enthalten, haben überdies den Vorteil, dass sie weniger zur Aggregation neigen, chemisch in großen Mengen synthetisiert werden können und, im Vergleich zum wildtyp Tridegin Polypeptid, eine verminderte Antigenizität aufweisen.

Weitere vorteilhafte, modifizierte Tridegin Polypeptide sind Tridegin Polypeptide, die kovalent mit Polyethylenglykol verknüpft sind. Die Reaktionsbedingungen für die Modifikation mit PEG sind z.B. in Cohen et al., Biochem. J., 357(3): 795-802 (2001) beschrieben. Das in die Modifizierungsreaktion eingesetzte Polyethylenglykol sollte ein Molekulargewicht von 500 Da bis 20000 Da, bevorzugt zwischen 1000 Da und 10000 Da, besonders bevorzugt zwischen 2000 Da und 5000 Da haben. Es sollte in einem molaren Verhältnis Polyethylenglykol : erfindungsgemäßes Polypeptid von zwischen 0,5:1 und 10:1, bevorzugt zwischen 0,8:1 und 4:1, besonders bevorzugt zwischen 1:1 und 2:1 eingesetzt werden. Diese modifizierten Polypeptide haben den Vorteil, nach Injektion in die Blutbahn eines Säugers weniger schnell abgebaut zu werden als das unmodifizierte Polypeptid.

Ein weiterer Gegenstand der Erfindung betrifft Verbindungen, die die oben beschriebenen Polypeptide enthalten, wie in den Ansprüchen definiert.

Fusionsproteine sind Proteine mit einem Anteil einer Aminosäuresequenz, die nicht vom Tridegin Polypeptid sondern zum Beispiel von einem anderen Protein stammt, von 5-500, vorzugsweise 5-400, 5-300, 5-200, 5-100, 5-50, vor allem 5-20 Aminosäuren (LaVallie and McCoy, Curr. Opin. Biotechnol. 6(5): 501-506 (1995)), ebenso Fusionsproteine mit einem Anteil einer Aminosäuresequenz, die nicht vom Tridegin Polypeptid sondern zum Beispiel von einem anderen Protein stammt, von mehr als 5, 6, 7, 8, 9, 10, 12, 14, 16, 18, 20, 25, 30 oder 50 Aminosäuren und weniger als 500, 450, 400, 350, 300, 250, 200, 150, 100, 75, 50 oder 40 Aminosäuren und sämtliche Permutationen in isolierter Form davon. Der Anteil einer Aminosäuresequenz, die vom Tridegin Polypeptid stammt, beträgt dabei vorzugsweise weniger als 50, 45, 40, 35, 30, 25 oder 20 Aminosäuren.

Beispiele solcher, von einem fremden Protein stammenden Aminosäuresequenzen, sind prokaryotische Peptid- und Polypeptidsequenzen, die z.B. von der Galaktosidase von *Escherichia coli* abgeleitet sein können. Weiterhin könnten auch virale Peptid- und Polypeptidsequenzen, wie z.B. vom Bakteriophagen N13 verwendet werden, um so Fusionsproteine für das dem Fachmann bekannte "Phage display"-Verfahren zu erzeugen (McCafferty et al., Nature 348(6301): 552-554 (1990)). Weiterhin können auch eukaryotische Polypeptidsequenzen, wie z.B. vom "Green fluorescent protein" (GFP, beschrieben in Prasher et al., Gene 111(2): 229-233 (1992)) verwendet werden, um so *in vivo* detektierbare, fluoreszente Fusionsproteine zu erzeugen. Es können auch Varianten von GFP (Tsien, Annu. Rev. Biochem. 67: 509-544 (1998)), ebenso das "Red fluorescent protein" verwendet werden. Weiterhin kann Glukosedehydrogenase und Polypeptidfragmente davon als Fusionspartner ausgeschlossen sein.

Gegenstand der Erfindung sind Peptid- und Polypeptidsequenzen für Fusionsproteine mit Peptiden, die die Aufreinigung der erfindungsgemäßen Polypeptide erleichtern, sogenannte Tags bzw.Tag, und somit zur Aufreinigung der erfindungsgemäßen Polypeptide verwendet werden können (siehe Nilsson et al., Protein Expr. Purif. 11(1): 1-16 (1997)). Tags an den erfindungsgemäßen Polypeptiden erlauben beispielsweise die hochaffine Absorption an eine Matrix, stringentes Waschen mit geeigneten Puffern, ohne den Komplex zwischen Fusionsprotein und Matrix in nennenswertem Maße zu eluieren und anschließend gezielte Elution des an die Matrix gebundenen Fusionsproteins. Beispiele für derartige Tags sind ein (His)₆-tag, wobei bereits fünf aufeinanderfolgende Histidine als Tag zur Aufreinigung verwendet werden können, ein Myc-tag, ein FLAG-tag, ein Chitin-bindender Tag, das Polypeptid Glutathion-Transferase (GST) oder das Polypeptid Maltose-binding-Protein (MBP). Dem Fachmann sind weitere Tags mit äquivalenter Funktion bekannt.

Weiter bevorzugte Beispiele für Peptid- und Polypeptidsequenzen für Fusionsproteine sind Peptide und Polypeptide, die die Sekretion der vorangehend beschriebenen Polypeptide aus einem Wirt vermitteln. Beispiele für solche Peptid- und Polypeptidsequenzen finden sich in Pines O. und Inouye M., supra; Rapoport TA., et al., supra, und Tjalsma H., et al., supra.

Ein weiterer Gegenstand der Erfindung betrifft ein Verfahren zur Herstellung der vorangehend beschriebenen Polypeptide. So können die erfindungsgemäßen Polypeptide mit gentechnischen oder peptid-chemischen Verfahren hergestellt werden.

Ein gentechnisches Verfahren zur Herstellung eines der genannten Polypeptide besteht z.B. darin, eine Nukleinsäure, die für eines der beschriebenen Polypeptide kodiert, in geeigneter Weise in prokaryotische oder eukaryotische Expressionsvektoren zu klonieren (Sambrook et al., "Molecular cloning: a laboratory manual" Secondedition, Cold Spring Harbor Laboratory Press (1989); Sambrook et al., "Molecular cloning: a laboratory manual" Third edition, Cold Spring Harbor Laboratory Press (2001)). Derartige Expressionsvektoren umfassen mindestens einen Promotor, mindestens ein Translationsinitiationssignal, mindestens eine Nukleinsäuresequenz, die für eines der erfindungsgemäßen Polypeptide kodiert, und ein Translationsterminationssignal im Falle von prokaryotischen Expressionsvektoren, im Falle von eukaryotischen Expressionsvektoren zusätzlich ein Transkriptionsterminierungssignal sowie ein Polyadenylierungssignal. Eine Nukleinsäure, die für eines der erfindungsgemäßen Polypeptide kodiert, kann beispielsweise Teil eines Vektors, wie eines Plasmid, eines Phagemids, eines Cosmids, eines BACs oder eines YACs, sein, insbesondere Teil eines prokaryontischen oder eukaryontischen Expressionsvektors (Sambrook et al., "Molecular Cloning: A Laboratory Manual", third edition, "Cold Spring Harbor Laboratory Press" (2001); Plasmide beschrieben in 1.3-1.29, Phagemids beschrieben in 3.42-3.52, Cosmide beschrieben in 4.1-4.10 und eukaryontische Expressionsvektoren in 17.83-17.111).

Weitere Beispiele für prokaryotische Expressionsvektoren sind für die Expression in *Escherichia coli* z.B. Expressionsvektoren basierend auf Promotoren, die von der T7 RNA Polymerase erkannt werden, wie in US 4,952,496 beschrieben, für die Expression in z.B. *Bacillus subtilis* die Expressionsvektoren, die von Le Grice S.F.J. beschrieben wurden in Methods in Enzymol. (1990) Vol. 185, Seiten 201-214, oder geeignet für die Sekretion bei *B. subtilis*, von Nagarajan V. beschrieben in Methods in Enzymol. (1990) Vol. 185, Seiten 214-223, für eukaryotische Expressionsvektoren für die Expression in *Saccharomyces cerevisiae* z.B. die Vektoren p426Met25 oder p526GALl (Mummberg et al. (1994) Nucl. Acids Res., 22, 5767-5768) oder die Vektoren beschrieben in Methods in Enzymol. (1990) Vol. 185, Seiten 297 - 329, beschrieben durch Mylin L.M., et al. (297-308), Price V.L., et al., (308-319) und Etcheverry T. (319-329) oder für die Sekretion in S. cerevisiae geeignete Vektoren wie beschrieben in Methods in Enzymol. (1990) Vol. 185, Seiten 408-440, beschrieben durch Brake A.J. (408-421) und Hitzeman R.A., et al. (421-440), für die Expression in Pichia Pastoris die Vektoren, wie z.B. in Cregg J.M. et al., Mol. Biotechnol. (2000) 16(1): 23-52 oder in "Pichia Protocols" D. R. Higgins und J.M. Cregg (ed.) Humana Press, Totowa, New Jersey beschrieben, für die Expression in anderen Hefen, z.B. in Hansenula polymorpha, die Vektoren beschrieben in Gellissen G., Appl. Microbiol. Biotechnol. (2000) 54(6):741-50, für die Expression in Insektenzellen z.B. *Baculovirus*-Vektoren wie in EP-B1-0 127 839 oder EP-B1-0 549 721 offenbart, und für die Expression in Säugerzellen z.B. die Vektoren Rc/CMV und Rc/RSV oder SV40-Vektoren oder die Vektoren beschrieben von Kaufman R.J. in Methods in Enzymol. (1990) Vol. 185, Seiten 487-512, welche alle allgemein erhältlich sind (für weitere relevante Expressionssysteme siehe auch Andersen D.C. und Krummen L., Curr. Opin. Biotechnol. (2002) 13(2):117-23).

Die molekularbiologischen Methoden zur Herstellung dieser Expressionsvektoren sowie die Methoden zum Einbringen der Expressionsvektoren in die Wirtszellen und auch die Bedingungen zur Kultivierung der transformierten Wirtszellen und schließlich die Bedingungen zur Induktion der Expression des gewünschten erfindungsgemäßen Polypeptids in den Wirtszellen sind dem Fachmann vertraut (Siehe auch Sambrook et al., supra). Beispiele für die gentechnische Herstellung erfindungsgemäßer Polypeptide sind in den Ausführungsbeispielen 1, 2 und 4 angegeben.

Die vorangehend beschriebenen Polypeptide können aber auch durch ein peptidchemisches Verfahren hergestellt werden, wie im Ausführungsbeispiel 3, also z.B. mit der allgemein bekannten Festphasensynthese, wie in Merrifield, J. Am. Che. Soc. 85: 2149 (1962) beschrieben. Techniken zur Synthese und Aufreinigung von Peptiden sind z.B. auch in Stewart and Young, "Solid Phase Peptide Synthesis" (Freeman, San Francisco, 1969) auf den Seiten 27-62 beschrieben, sowie in US 4,269,827.

Ein anderer Gegenstand der Erfindung ist die Verwendung eines der oben genannten Polypeptide als Inhibitoren von Transglutaminasen, insbesondere von Faktor XIIIa, vor allem von humanem Faktor XIIIa. Die oben genannten Polypeptide haben die Eigenschaft die vom Faktor XIIIa katalysierte Freisetzung von Ammonium-Ionen, die bei der von Faktor XIIIa katalysierten Reaktion von einem spezifischen Peptidsubstrat mit Glycinethylester freigesetzt werden, wie z.B. im Berichrom^{®} assay (Fa. Dade Behring GmbH, Marburg), zu inhibieren. Eine derartige inhibitorische Wirkung der oben genannten Polypeptide kann, zum Beispiel, im Berichrom^{®} assay nachgewiesen werden, wie in den Ausführungsbeispielen 1 bis 4 beschrieben.

Des weiteren können die erfindungsgemäßen Polypeptide die zum humanen Faktor XIIIa homologen Säugerproteine inhibieren, z.B.das mit dem humanen Faktor XIII homologe Protein aus *Rattus norvegicus,* bei dem 617 von 732 Aminosäuren identisch sind (84%), und 689 von 732 Aminosäuren verwandt (93%).

Neben ihrem inhibitorischen Effekt auf den Faktor XIIIa inhibieren die oben genannten Polypeptide auch weitere Transglutaminasen, z.B. die in den Keratinozyten exprimierte Transglutaminase 1, die an der Bildung der Epidermis beteiligte Transglutaminase 3, die im Samenleiter bei der Quervernetzung von Proteinen und der Konjugation von Polyaminen beteiligte Transglutaminase 4, sowie die bei der Verhornung von Keratinozyten beteiligte Transglutaminase 5, und damit alle sechs bislang beschriebenen Transglutaminasen des menschlichen Proteoms.

Eine weiterer Aspekt besteht in der Verwendung der oben genannten Polypeptide zur Prävention und Therapie von Thrombosen. Indem die Polypeptide den Faktor XIIIa inhibieren, inhibieren sie auch die Bildung von Quervernetzungen von Fibrinpolymeren. Damit wird die Bildung von "harten" Blutthromben, die resistent gegen den Abbau durch fibrinolytische Enzyme sind, inhibiert.

Die erfindungsgemäßen Polypeptide ermöglichten z.B. eine schnellere Lyse von humanen Blutthromben und inhibierten auch das Einsetzen der Blutgerinnung, wie im Ausführungsbeispiel 5 beschrieben. Sie eignen sich somit zur Herstellung eines Arzneimittels zur Prävention und Therapie von Thrombosen.

Ein weiterer Gegenstand der Erfindung betrifft ein Arzneimittel enhaltend ein erfindungsgemäßes Polypeptid und mindestens einen galenischen Hilfsstoff. Die erfindungsgemäßen Polypeptide sind potente Transglutaminase-Inhibitoren, weisen aber nur ein geringes Maß an Toxitität auf und können darum zur Herstellung von Arzneimitteln besonders gut verwendet werden.

Der Begriff "galenischer Hilfsstoff' bedeutet erfindungsgemäß jedes inerte, nichttoxische, feste oder flüssige Füll-, Verdünnungs-, oder Verpackungsmaterial, solange es nicht ungebührend nachteilhaft mit dem erfindungsgemäßen Polypeptid oder dem Patienten reagiert. Flüssige galenische Hilfsstoffe sind zum Beispiel steriles Wasser, physiologische Kochsalzlösung, Zuckerlösungen, Ethanol und/oder Öle. Galenische Hilfsstoffe zur Herstellung von Tabletten und Kapseln können zum Beispiel Bindemittel und Füllmaterial enthalten.

Ein weiterer Gegenstand der Erfindung ist ein Kombinationspräparat enthaltend ein erfindungsgemäßes Polypeptid sowie mindestens einen pharmazeutischen Wirkstoff.

Eine bevorzugte Ausführungsform der Erfindung besteht in einem Kombinationspräparat enthaltend mindestens eines der erfindungsgemäßen Peptide sowie einen weiteren Wirkstoff in Form eines Antikoagulanz. Antikoagulanzien fördern entweder die Lyse von Blutthromben oder inhibieren die Bildung von Blutthromben. Beispiele sind thrombolytische Wirkstoffe, also Wirkstoffe die den Abbau von aktivem Thrombin oder dem Prothrombin fördern, fibrinolytische Wirkstoffe, also Wirkstoffe, die den Abbau von polymerem Fibrin fördern, oder fibrinogenolytische Wirkstoffe, also Wirkstoffe, die den Abbau von Fibrinogen fördern. Bevorzugte Antikoagulanzien sind Aktivatoren von Plasmin oder Plasminogen oder Inhibitoren von Thrombin, Faktor Xa oder Inhibitoren der Blutplättchen-Aggregation .

Besonders bevorzugte Antikoagulanzien, die in Kombinationspräparaten gemeinsam mit den erfindungsgemäßen Peptiden verwendet werden können, sind Acetylsalicylsäure, Heparin, niedermolekulares Heparin, Heparinoid, Hirudin, Bivalirudin, Melagatran, Abciximab, Eptifibatide, Gewebe Plasminogen Aktivator (tPA), Streptokinase, Staphylokinase, Urokinase, Eminase, Hementin und/oder Plasmin.

Acetylsalicylsäure wirkt u.a. als Inhibitor der Blutplättchen-Aggregation. Heparin ist ein körpereigenes polyanionisches Polysaccharid mit einem Molekulargewicht von 6000 Da bis 30000 Da und erhöht die Wirksamkeit des körpereigenen Antithrombin III. Niedermolekulares Heparin wird durch begrenzten Abbau von Heparin gewonnen und hat ein Molekulargewicht von 4000 Da bis 6000 Da. Hirudin ist z.B. in EP 0347376 und EP 0501821 beschrieben. Mit Hirudin bezeichnet man eine Familie von homolgen Polypeptiden aus Blutegeln, die Thrombin inhibieren und die Blutgerinnung inhibieren. Bivalirudin ist ein Thrombin inhibierendes Peptid (Kelly et al., Proc. Natl. Acad. Sci USA, 89, 6040-6044 (1992). Melagatran ist ein Thrombin inhibierendes Peptid Mimetikum (Thromb Haemost 79(1): 110-118 (1998)). Abciximab ist ein Antikörper und Eptifibabite ein Peptid; beide binden GP IIb/IIIb, das "platelet glycoprotein" IIb/IIIb, und hemmen die Blutplättchen-Aggregation. Hementin ist z.B. beschrieben in WO 91/15576, wird in verschiedenen Blutegeln gefunden, baut Fibrinogen ab und verhindert damit die Blutgerinnung. Ebenso führen Plasmin oder Eminase zum Fibrinabbau, während Streptokinase, Urokinase, Staphylokinase und der Gewebe Plasminogen Aktivator (tPA) das Plasminogen aktivieren und durch die Generierung von aktiven Plasmin zum Fibrinabbau führten.

Ein besonderer Vorteil der Kombination der erfindungsgemäßen Polypeptide mit den Antikoagulanzien und gegebenenfalls einem weiteren pharmazeutischen Wirkstoff, besteht darin, dass Blutthromben durch die Kombination der Wirkstoffe auf synergistische Weise schneller aufgelöst werden als durch einen Wirkstoff allein. Besonders die Kombination mit fibrinolytischen Agenzien, wie z.B. Urokinase und Gebwebe Plasminogen Aktivator (tPA), bewirkte eine verminderte Stabilität und eine beschleunigte Auflösung von Blutthromben, wie im Ausführungsbeispiel 5 detailliert beschrieben ist.

### Beschreibung der Figuren und Sequenzen:

Figur 1: Karte des Expressionsplasmids pET22b-14 (A) und Angabe der für das Tridegin Polypeptid kodierenden Sequenz (B). Nummerierung der Basen gemäß der Plasmidkarte.
Figur 2: Reinigung von rekombinantem wildtyp Tridegin Polypeptid, untersucht mittels SDS-PAGE (10% Gel)
Spur 1 ist *E* .*coli*-Totallysat vor Auftrag auf die Ni-NTA-Säule.
Spur 2-7 sind Fraktionen der Elution mit Imidazol. Alle Proben wurden mit SDS-Probenpuffer versetzt und 5 min. bei 95° C inkubiert.
Figur 3: Inhibitorische Wirkung des rekombinanten, gereinigten Tridegin Polypeptides auf den Faktor XIIIa im Berichrom^{®}-Assay.
Als Kontroll-Substanz wurde Cerulenin (der Fa. Calbiochem) verwendet.
Figur 4: Schematische Darstellung eines Thrombelastogrammes.
Die für die beschriebenen Experimente relevanten Parameter sind CT (clotting time, Gerinnungszeit), MCF (maximum clot firmness, maximale Thrombus Festigkeit) und LT (lysis time, Fibrinolyse Zeit).
Figur 5: Thrombelastogramme von Citrat-Vollblut in Ab- (A, C, E) und Anwesenheit (B, D, F) von rekombinantem Tridegin.
Alle Ansätze enthalten Citrat-Vollblut (300 µl), Ca²⁺ (20 µl Starteg-Reagenz) und Thromboplastin-Phospholipid (10 µl Integ-Reagenz).
B: + Transglutaminase Inhibitor der SEQ ID No.1 (10 µM)
C: + Urokinase (25 E)
D: + Transglutaminase Inhibitor der SEQ ID No.1 (10 µM) und Urokinase (25 E)
E: + tPA (40,5 ng)
F: + Transglutaminase Inhibitor der SEQ ID No.1 (10 µM) und tPA (40,5 ng)

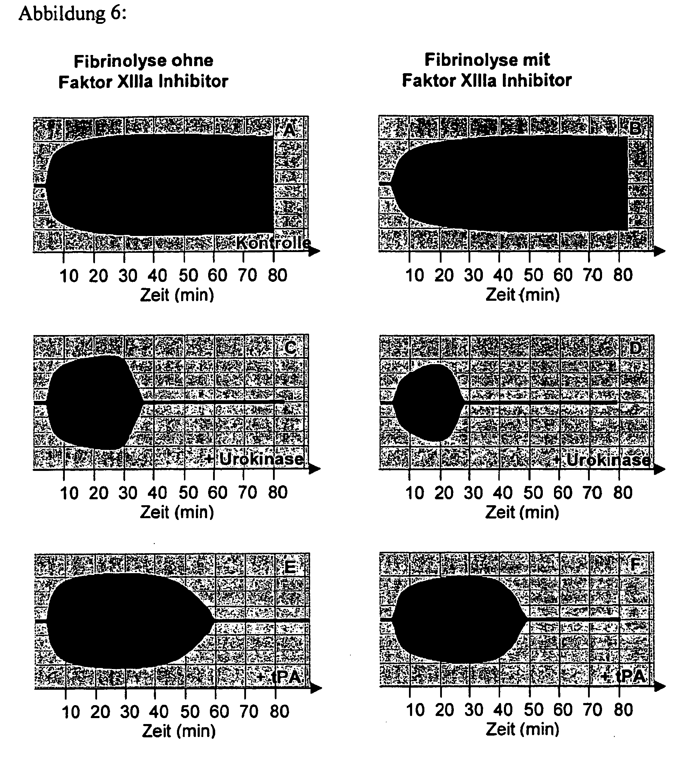

Figur 6: Thrombelastogramme von Citrat-Vollblut in Ab- (A, C, E) und Anwesenheit (B, D, F) von SEQ ID No. 25
Alle Ansätze enthalten Citrat-Vollblut (300 µl), Ca²⁺ (20 µl Starteg-Reagenz) und Thromboplastin-Phospholipid (10 µl Integ-Reagenz).
B: + Transglutaminase Inhibitor der SEQ ID No. 25 (20 µM)
C: + Urokinase (25 E) + inaktives Kontroll-Peptid (Acetyl-Adhesin (1025-1044) Amid der Fa. *Bachem*) (20 µM)
D: + Transglutaminase Inhibitor der SEQ ID No. 25 (20 µM) und Urokinase (25 E)
E: + tPA (40,5 ng) + inaktives Kontroll-Peptid (20 µM)
F: + Transglutaminase Inhibitor der SEQ ID No. 25 (20 µM) und tPA (40,5 ng)

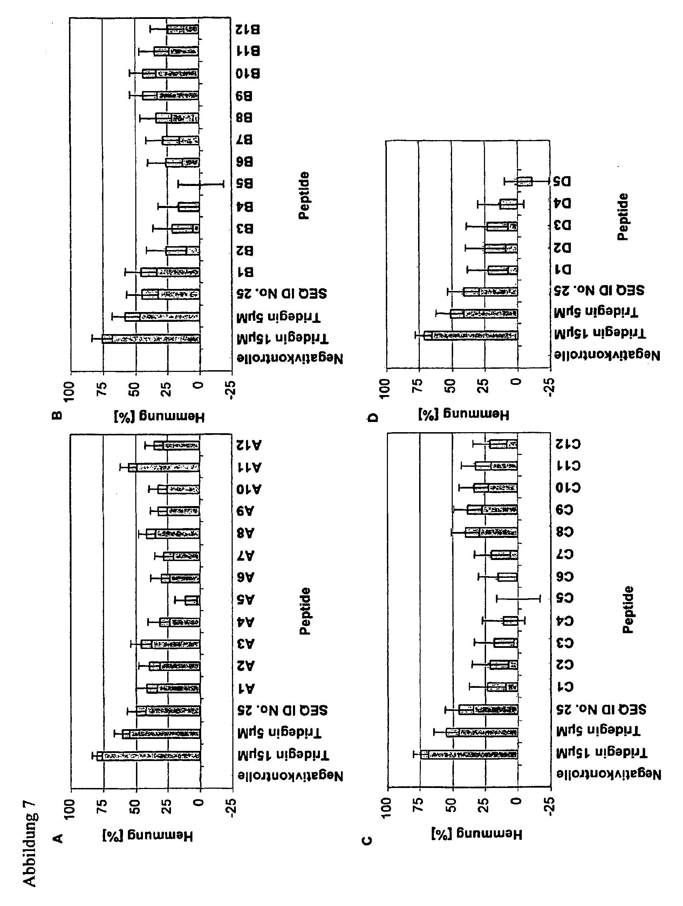

Figur 7: Inhibitorische Wirkung der von Tridegin abgeleiteten Peptide und deren Varianten auf den Faktor XIIIa im Berichrom^{®}-Assay (A - D). Als Kontrolle wurde rekombinantes, gereinigtes Tridegin aus *E. coli* in den angegebenen Konzentrationen und das Peptid 25 (SEQ ID No 25; ~7.27 µM Endkonzentration im Assay) verwendet. Die Sequenzen der verwendeten Peptide sind unter (E) aufgeführt. Die Fehlerbalken beziehen sich auf den Standart-Fehler (n=3).

| | | |
|---|---|---|
| A1: | PMDDIYQRPVEFPNLPLKPR | SEQ ID No. 25 |
| A2: | PMDDIYQRPVEFPNLPLKPA | SEQ ID No. 67 |
| A3: | PMDDIYQRPVEFPNLPLKAR | SEQ ID No. 68 |
| A4: | PMDDIYQRPVEFPNLPLAPR | SEQ ID No. 69 |
| A5: | PMDDIYQRPVEFPNLPAKPR | SEQ ID No. 70 |
| A6: | PMDDIYQRPVEFPNLALKPR | SEQ ID No. 71 |
| A7: | PMDDIYQRPVEFPNAPLKPR | SEQ ID No. 72 |
| A8: | PMDDIYQRPVEFPALPLKPR | SEQ ID No. 73 |
| A9: | PMDDIYQRPVEFANLPLKPR | SEQ ID No. 74 |
| A10: | PMDDIYQRPVEAPNLPLKPR | SEQ ID No. 75 |
| A11: | PMDDIYQRPVAFPNLPLKPR | SEQ ID No. 76 |
| A12: | PMDDIYQRPAEFPNLPLKPR | SEQ ID No. 77 |
| B1: | PMDDIYQRAVEFPNLPLKPR | SEQ ID No. 78 |
| B2: | PMDDIYQAPVEFPNLPLKPR | SEQ ID No. 79 |
| B3: | PMDDIYARPVEFPNLPLKPR | SEQ ID No. 80 |
| B4: | PMDDIAQRPVEFPNLPLKPR | SEQ ID No. 81 |
| B5: | PMDDAYQRPVEFPNLPLKPR | SEQ ID No. 82 |
| B6: | PMDAIYQRPVEFPNLPLKPR | SEQ ID No. 83 |
| B7: | PMADIYQRPVEFPNLPLKPR | SEQ ID No. 84 |
| B8: | PADDIYQRPVEFPNLPLKPR | SEQ ID No. 85 |
| B9: | AMDDIYQRPVEFPNLPLKPR | SEQ ID No. 86 |
| B10: | MDDIYQRPVEFPNLPLKPR | SEQ ID No. 48 |
| B 11: | DDIYQRPVEFPNLPLKPR | SEQ ID No. 49 |
| B12: | DIYQRPVEFPNLPLKPR | SEQ ID No. 50 |
| C1: | IYQRPVEFPNLPLKPR | SEQ ID No. 61 |
| C2: | YQRPVEFPNLPLKPR | SEQ ID No. 52 |
| C3: | QRPVEFPNLPLKPR | SEQ ID No. 3 |
| C4: | RPVEFPNLPLKPR | SEQ ID No. 54 |
| C5: | PVEFPNLPLKPR | SEQ ID No. 55 |
| C6: | VEFPNLPLKPR | SEQ ID No. 56 |
| C7: | EFPNLPLKPR | SEQ ID No. 57 |
| C8: | PMDDIYQRPVEFPNLPLKP | SEQ ID No. 58 |
| C9: | PMDDIYQRPVEFPNLPLK | SEQ ID No. 59 |
| C10: | PMDDIYQRPVEFPNLPL | SEQ ID No. 60 |
| C 12: | PMDDIYQRPVEFPNLP | SEQ ID No. 61 |
| D1: | PMDDIYQRPVEFPNL | SEQ ID No. 62 |
| D2: | PMDDIYQRPVEFPN | SEQ ID No. 63 |
| D3: | PMDDIYQRPVEFP | SEQ ID No. 64 |
| D4: | PMDDIYQRPVE | SEQ ID No. 65 |
| D5: | PMDDIYQRPV | SEQ ID No. 66 |

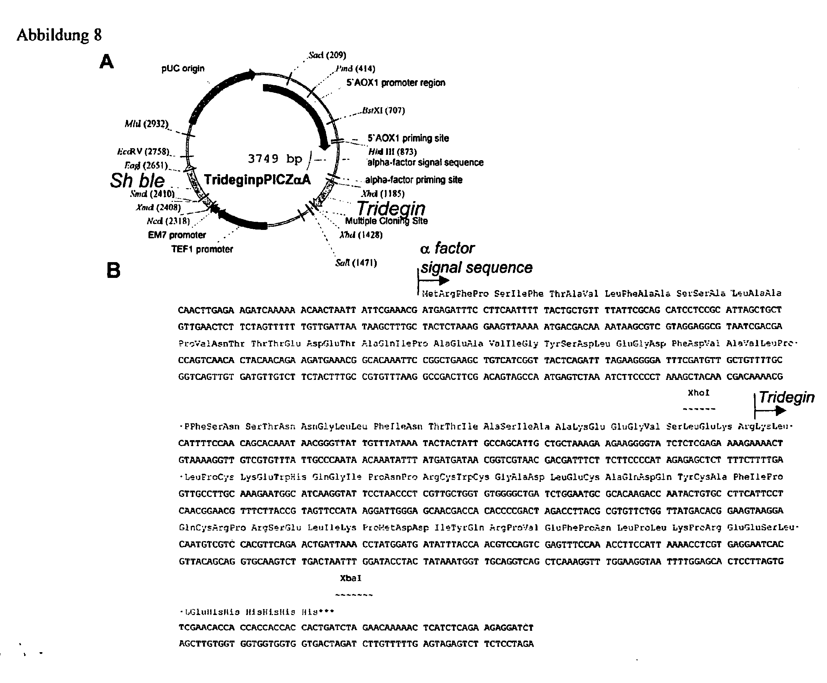

Figur 8: Karte des Expressionsplasmids TrideginpPICZαA (A) und Angabe der für das Tridegin Polypeptid kodierenden Sequenz (B). Nummerierung der Basen gemäß der Plasmidkarte.

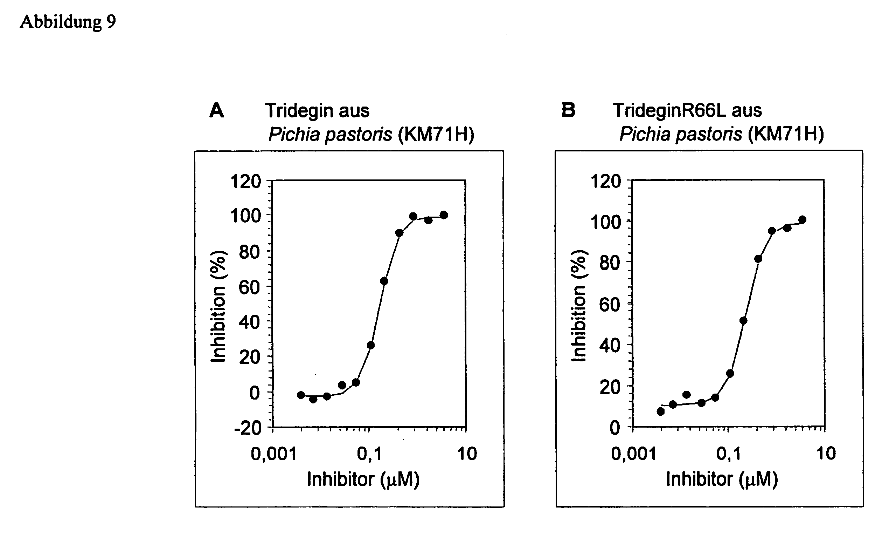

Figur 9: Inhibitorische Wirkung des rekombinanten, gereinigten Tridegin Polypeptides (A) oder der Variante TrideginR66L (B) aus *Pichia pastoris* (KM71H), auf den Faktor XIIIa im Berichrom®-Assay.

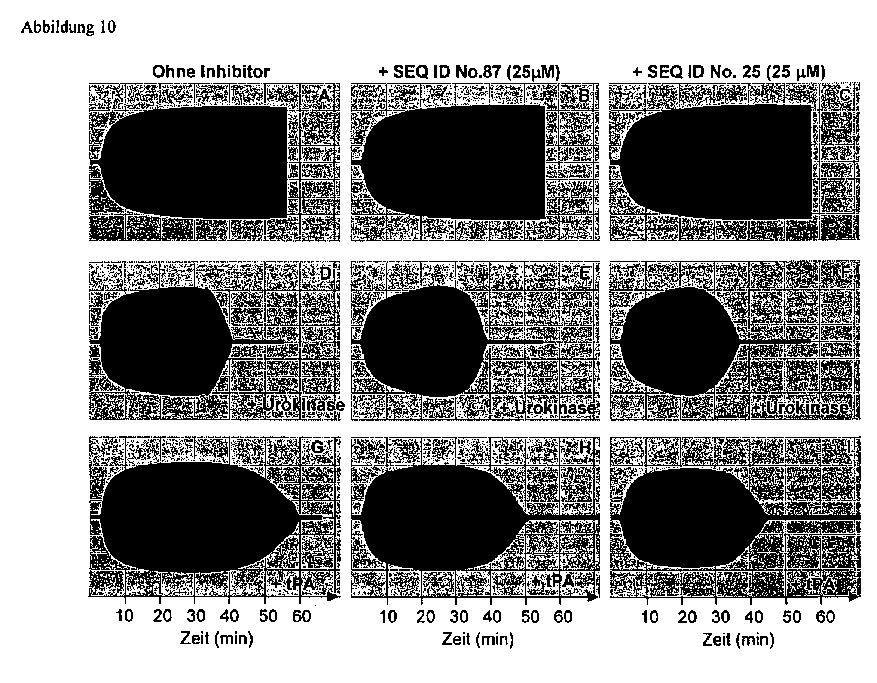

Figur 10: Thrombelastogramme von Citrat-Vollblut in Ab- (A, D, G) und Anwesenheit (B, C, E, F, H, I) von SEQ ID No. 87 oder SEQ ID No. 25 (beide 25 µM). Alle Ansätze enthalten Citrat Vollblut (300 µl) Ca²⁺ (20 µl Starteg Reagenz, und Thromboplastin-Phospholipid (10 µl Integ-Reagenz).
B: + Transglutaminase Inhibitor (TI) der SEQ ID No. 87 (25 µM)
C: + TI der SEQ ID No. 25 (25 µM)
D: + Urokinase (25 E)
E: + TI der SEQ ID No. 87 (25 µM) + Urokinase (25 E)
F: + TI der SEQ ID No. 25 (25 µM) + Urokinase (25 E)
G: + tPA (40,5 ng)
H: + TI der SEQ ID No. 87(25 µM) + tPA (40,5 ng)
I: + TI der SEQ ID No. 25 (25 µM) + tPA (40,5 ng)

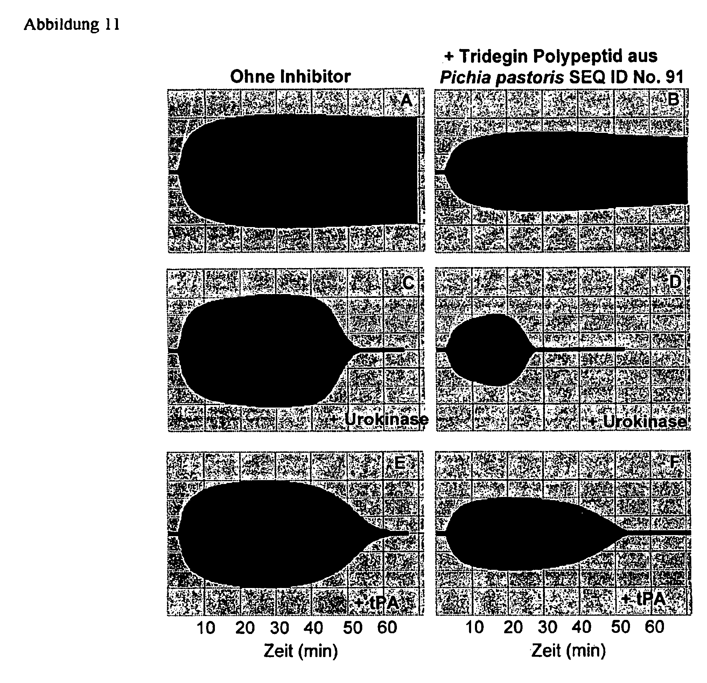

Figur 11: Thrombelastogramme von Citrat-Vollblut in Ab- (A, C, E) und Anwesenheit (B, D, F) von rekombinantem, gereingtem TrideginR66L aus *Pichia pastoris* (codiert von SEQ ID No. 91) . Alle Ansätze enthalten Citrat Vollblut (300 µl) Ca²⁺ (20 µl Starteg Reagenz, und Thromboplastin-Phospholipid (10 µl Integ-Reagenz).
B: + Transglutaminase Inhibitor (TI) der SEQ ID No. 91 (5 µM)
C: + Urokinase (25 E)
D: + TI der SEQ ID No. 91 (5 µM) + Urokinase (25 E)
E: + tPA (40,5 ng)
F: + TI der SEQ ID No. 91 (5 µM) + tPA (40,5 ng)

SEQ ID No. 1 zeigt das wildtyp Tridegin Polypeptid.

SEQ ID No. 2 bis SEQ ID Nr. 26 zeigen je 20 Aminosäure lange Peptide von SEQ ID No. 1.

SEQ ID No. 27 bis SEQ ID No. 46 zeigen für die Mutangenese des Tridegin Polypeptides verwendete Oligonukleotide.

SEQ ID Nr. 47 zeigt ein 16 Aminosäure langes Peptid von SEQ ID Nr. 1, SEQ ID No. 92 zeigt ein 15 Aminosäuren langes Peptid von SEQ ID No.1.

SEQ ID No. 48 bis SEQ ID No. 88 zeigen verkürzte Peptide und Peptid-Varianten.

SEQ ID No. 89 zeigt ein 15 Aminosäuren langes Peptid.

SEQ ID No. 90 und SEQ ID No. 91 zeigen für die Expression in *Pichia pastoris* verwendete codierende DNA-Sequenzen.

### Ausführungsbeispiele

### Beispiel 1: Expression und Reinigung von rekombinantem Tridegin Polypeptid SEQ ID NO:1) aus Escherichia coli

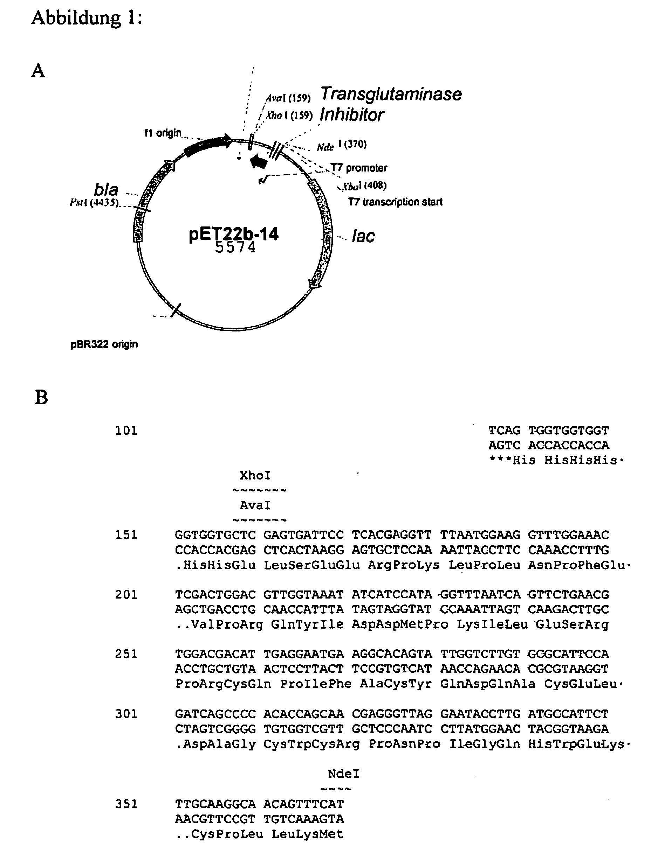

Das Expressionsplasmid pET22b-14, das die codierende Sequenz für das rekombinante Tridegin Polypeptid enthält, ist in Abbildung 1 dargestellt. Das Plasmids wurde nach gängigen Verfahren in den *Escherichia coli* -Expressionsstamm Origami® B (DE3) (der Fa. Novagen, Best. Nr. 70837 ) transferiert und in LB-Flüssigmedium mit Ampicillin (100 µg/ml), Kanamycin und Tetracyclin (je 5 µg/ml) kultiviert. Der Expressionsstamm BL21(DE3) (der Fa. Novagen) lieferte ähnliche Ergebnisse und kann ebenso für die Expression der modifizierten Tridegin Polypeptide verwendet werden. Die Hauptkultur wurde bei 37°C und 220 rpm geschüttelt, bis eine OD600 von 0,7 erreicht wurde. Bei einer Zelldichte von 0,7-0,9 OD600 /ml wurde die Kultur zur Induktion der Genexpression mit 2 mM IPTG/ml versetzt und für weitere 4h bei 37°C bei 200-240 rpm geschüttelt. Die Zellen wurden durch anschliessende Zentrifugation geerntet (15 min, 5825 x g).

Das Zellsediment wurde in 1:10 mit Reinst-Wasser verdünntem BugBuster 10x Protein Extraction Reagent (der Fa. Novagen) resuspendiert und zum Aufschluss mit Benzonase (der Fa. Novagen) und Protease-Inhibitor-Cocktail (Complete® ohne EDTA, der Fa. Roche Diagnostics GmbH ) für 10 - 20 min unter Schütteln bei 4°C inkubiert. Durch anschließende Zentrifugation bei 16,000 x g für 20 min bei 4°C wurde der Überstand gewonnen und mit dem gleichen Volumen an Lysispuffer (50 mM NaH₂PO₄ pH 8.0, 300 mM NaCl, 10 mM Imidazol) versetzt. Die so erhaltene Proteinsuspension wurde bei 4°C über Nacht gelagert. Zur Reinigung wurden 3 ml Nickel-NTA-Agarose (der Fa. Quiagen) in eine Leer-Säule gepackt und mit 5 Säulenvolumina Lysispuffer equilibriert. Die Proteinsuspension wurde auf die Säule aufgetragen (ohne Pumpe, Fluß durch Schwerkraft) und anschließend mit Waschpuffer (50 mM NaH₂PO₄ pH 8.0, 300 mM NaCl, 20 mM Imidazol) gewaschen (10 Säulenvolumina). Die Elution erfolgte mittels Elutionspuffer (2-3 Säulenvolumina) (50 mM NaH₂PO₄, 300 mM NaCl, 250 mM Imidazol, pH 8). Fraktionen wurden gesammelt und mit SDS-PAGE auf die Anwesenheit von Transglutaminase-Inhibitor geprüft (siehe Abbildung 2). Die Ausbeute betrug 20 mg rekombinantes Tridegin Polypeptid pro Liter Expressionskultur bei einer Reinheit von > 90 %. Die Fraktionen, die das rekombinante Tridegin Polypeptid enthielten, wurden vereinigt und extensiv gegen 50 mM NaH₂PO₄ pH 8.0, 300 mM NaCl dialysiert (2x gegen 21). Anschließend wurde die inhibitorische Aktivität des gereinigten Proteins auf Faktor XIIIa getestet. Als Testverfahren wurde der Berichrom®-Assay (der Fa. Dade Behring) verwendet.

### Durchführung des Berichrom® Assays:

Der Assay beruht darauf, daß Faktor XIII durch in den Reagentien vorhandenes Thrombin zu Faktor XIIIa aktiviert wird. Faktor XIIIa verknüpft ein spezifisches Peptidsubstrat mit Glycinethylester unter Freisetzung von Ammonium Ionen. Diese werden in einer parallel ablaufenden enzymatischen Reaktion bestimmt. Gemessen wurde die Abnahme von NADH über die Extinktion bei 340 nm. Peptide lagen für die Messung in 50% Acetonitril in einer Stamm-Konzentration von 5 mM vor. Die vom Hersteller gelieferten NADH- und Nachweis-Reagentien wurden in 3 ml Wasser, und das Aktivator-Reagenz anschließend in 3 ml NADH-Reagenz gelöst. Aktivator- und Nachweisreagenz wurden 1:1 zum Gebrauch gemischt. Zur Messung im Mikrotiterplattenformat wurden 100 µl Probe (Inhibitor, bzw Kontrollpuffer), 25 µl Faktor XIII (10 E/ml) und 150 µl Gebrauchsreagenz gemischt. Die Messung erfolgte kontinuierlich über 20 min bei 37°C in einem Mikrotiterplatten-Photometer bei 340 nm. Zur Auswertung wurden die Differenzen der Messwerte nach 16 und 20 min verglichen.

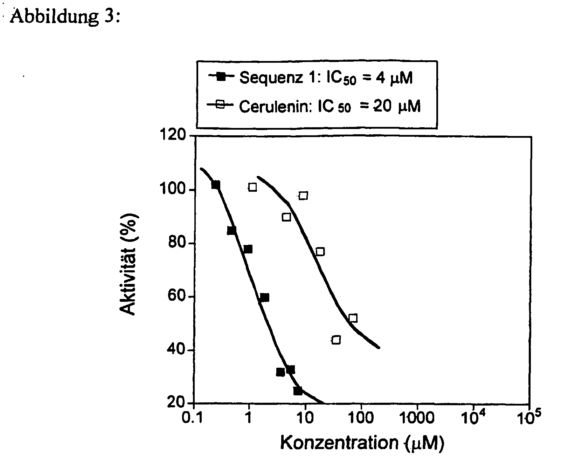

Die Messung ergab für den gereinigten Transglutaminase Inhibitor einen IC₅₀ von 2-4 µM (s. Abbildung 3)

### Beispiel 2: Expression und Reinigung von modifizierten Trideginen aus Escherichia coli

Modifizierte Tridegine wurden durch gezielte Mutagenese des für das wildtyp Tridegin Polypeptid kodierenden Expressionsplasmides erzeugt. Die Mutagenese wurde durch PCR mit den QuikChange Reagenzien (der Fa. Stratagene) laut Anleitung des Herstellers durchgeführt. Die Oligonukleotide SEQ ID Nr. 27 bis SEQ ID Nr. 40 und die jeweiligen revers-komplementären Sequenzen wurden für die Mutagenese verwendet.

Die DNA-Sequenzen der erhaltenen Mutanten wurden durch Sequenzierung überprüft. Die jeweilige codierende Sequenz im Plasmid pET22b wurde nach gängigen Verfahren in den *Escherichia coli* -Expressionsstamm Origami^{®} B(DE3) (der Fa. Novagen) transferiert und in LB-Flüssigmedium mit Ampicillin, Kanamycin und Tetracyclin wie oben bereits beschrieben kultiviert. Zusätzlich gefundene, spontan aufgetretene Doppelmutanten wurden auch exprimiert und gereinigt. Die Expression, Reinigung und Aktivitätsbestimmung erfolgte wie in Beispiel 1 genannt. Folgende Aktivitäten wurden für die einzelnen modifizierten Tridegine gemessen (Tabelle 2). Die Bezeichnung der modifizierten Tridegine erfolgte nach dem Schema XnY.

Dabei bezeichnet X die Aminosäure, die durch Mutagenese verändert wurde, n definiert die Position dieser Aminosäure in der Polypeptidkette und Y bezeichnet die nach der Mutagenese vorhandene Aminosäure.

**Tabelle 2: Inhibitorische Wirkung von modifizierten Trideginen auf den Faktor XIIIa im Berichrom^{®}-Assay.**

| Variante | Relative inhibitorische Wirkung (%) |
|---|---|
| wildtyp Polypeptid | 100 |
| K02A | 113 |
| K07A | 90 |
| H10A | 87 |
| G12A | 97 |
| L24A | 92 |
| Y31A | 69 |
| F34A | 96 |
| R39A | 49 |
| I45A | 115 |
| M48A | 91 |
| D50A | 53 |
| D50A, P55L | 84 |
| F58A | 60 |
| N60A | 68 |
| P65A | 51 |

Zur Erzeugung der oben genannten Varianten wurden die folgenden Oligonukleotide verwendet:

| Oligonukleotid | SEQ ID No. | Variante |
|---|---|---|
| 5'-ccttgatgccattctttgcaaggcaacagtgccatatgtatatctccttc | 33 | K02A |
| 5'-catatgaaactgttgccttgcgcagaatggcatcaaggtattcctaaccc | 34 | K07A |
| 5'-cgagggttaggaataccttgagcccattctttgcaaggcaacag | 31 | H10A |
| 5'-cttgcaaagaatggcatcaagctattcctaaccctcgttgctggtg | 30 | G12A |
| 5'-gtattggtcttgtgcgcattccgcatcagccccacaccag | 35 | L24A |
| 5'-gaggaatgaaggcacaagcttggtcttgtgcgcattccagatc | 40 | Y31A |
| 5'-gtggacgacattgaggaatggcggcacagtattggtcttgtgc | 28 | F34A |
| 5'-ggtttaatcagttctgaacgtggagcacattgaggaatgaaggcacagtattg | 39 | R39A |
| 5'-ttggtaaatatcatccataggtttagccagttctgaacgtggacgacattgagg | 32 | I45A |
| 5'-cgactggacgttggtaaatatcatccgcaggtttaatcagttctgaacgtggacg | 36 | M48A |
| 5'-ctcgactggacgttggtaaatagcatccataggtttaatcagttctgaacgtgg | 27 | D50A |
| 5'-cgaggttttaatggaaggtttggagcctcgactggacgttggtaaatatcatcc | 29 | F58A |
| 5'-cctcacgaggttttaatggaagggctggaaactcgactggacg | 37 | N60A |
| 5'-gtggtgctcgagtgattcctcacgagcttttaatggaaggtttgg | 38 | P65A |

Die relative inhibitorische Wirkung (%) wurde bei einer Endonzentration der Variante von 5,45 µM bestimmt. Die inhibitorische Wirkung des rekombinanten Tridegin Polypeptides wurde auf 100 % (bei 5,45 µM) normiert.

### Beispiel 3: Inhibitorische Wirkung von Fragmenten des Tridegin Polypeptides

25 Peptide mit einer Länge von 20 Aminosäuren wurden auf Basis des rekombinanten Tridegin Polypeptides nach einem gängigen Verfahren der Peptidsynthese chemisch synthetisiert (von der Fa. Pepscan, Lelystad, NL). Die Peptide tragen N-terminal eine Acetylgruppe und C-terminal entsprechend eine Amidgruppe. Die Sequenzen wurden so gewählt, dass diese
a) die gesamte Sequenz 1 abdecken und
b) mit jeweils in 18 Aminosäurenresten überlappen (siehe Tabelle 3, Sequenzen 2-26).

Die relative inhibitorische Wirkung (%) wurde mittels des oben beschriebenen Berichrom^{®} -Assays bei einer Endkonzentration der Peptide von 7,27 µM bestimmt. Die inhibitorische Wirkung des rekombinanten Tridegin Polypeptides wurde bei einer Endkonzentration von 7,27 µM auf 100 % normiert.

**Tabelle 3: Inhibitorische Wirkung von Peptiden des rekombinanten Tridegins auf den Faktor XIIIa im Berichrom^{®}-Assay.**

| Sequenz (Sequenz Nr.) | Relative inhibitorische Wirkung (%) |
|---|---|
| MKLLPCKEWHQGIPNPRCWC (SEQ ID NO. 2) | 0,00 |
| LLPCKEWHQGIPNPRCWCGA (SEQ ID NO.3) | 5,88 |
| PCKEWHQGIPNPRCWCGADL (SEQ ID NO.4) | 9,80 |
| KEWHQGIPNPRCWCGADLEC (SEQ ID NO.5) | 1,96 |
| WHQGIPNPRCWCGADLECAQ (SEQ ID NO.6) | 9,80 |
| QGIPNPRCWCGADLECAQDQ (SEQ ID NO.7) | 13,73 |
| IPNPRCWCGADLECAQDQYC (SEQ ID NO.8) | 13,73 |
| NPRCWCGADLECAQDQYCAF (SEQ ID NO.9) | 0,00 |
| RCWCGADLECAQDQYCAFIP (SEQ ID NO.10) | 5,88 |
| WCGADLECAQDQYCAFEPQC (SEQ ID NO.11) | 0,36 |
| GADLECAQDQYCAFIPQCRP (SEQ ID NO. 12) | 2,49 |
| DLECAQDQYCAFIPQCRPRS (SEQ ID NO.13) | 7,96 |
| ECAQDQYCAFIPQCRPRSEL (SEQ ID NO.14) | 6,30 |
| AQDQYCAFIPQCRPRSELIK (SEQ ID NO.15) | 3,32 |
| DQYCAFIPQCRPRSELIKPM (SEQ ID NO.16) | 14,76 |
| YCAFEPQCRPRSELIKPMDD (SEQ ID NO.17) | 12,27 |
| AFIPQCRPRSELIKPMDDIY (SEQ ID NO.18) | 13,27 |
| IPQCRPRSELIKPMDDIYQR (SEQ ID NO.19) | 0,0 |
| QCRPRSELIKPMDDIYQRPV (SEQ ID NO.20) | 11,11 |
| RPRSELIKPMDDIYQRPVEF (SEQ ID NO.21) | 19,40 |
| RSELIKPMDDIYQRPVEFPN (SEQ ID NO.22) | 8,64 |
| ELIKPMDDIYQRPVEFPNLP (SEQ ID NO.23) | 24,01 |
| IKPMDDIYQRPVEFPNLPLK (SEQ ID NO.24) | 43,52 |
| PMDDIYQRPVEFPNLPLKPR (SEQ ID NO.25) | 42,14 |
| DDIYQRPVEFPNLPLKPREE (SEQ ID NO.26) | 35,58 |

Die drei C-terminalen Peptide (SEQ ID NO:24, 25, 26) wurden nach HPLC Aufreinigung nochmals getrennt auf ihre inhibitorische Wirkung auf den Faktor XIIIa mittels Berichrom®-Assay gemessen. Folgende IC₅₀-Werte wurden gemessen:
- SEQ ID No. 24: IC₅₀: 7 µM
- SEQ ID No. 25: IC₅₀: 4 µM
- SEQ ID No. 26: IC₅₀: 5 µM

Zur Bestimmung der minimalen Länge wurden 20 Peptide (acetyliert und amidiert) nach gängigen Verfahren synthetisiert die vom C- oder N-Terminus um jeweils eine Aminosäure verkürzt wurden.

| | | |
|---|---|---|
| B10 | MDDIYQRPVEFPNLPLKPR | SEQ ID No. 48 |
| B11 | DDIYQRPVEFPNLPLKPR | SEQ ID No. 49 |
| B12 | DIYQRPVEFPNLPLKPR | SEQ ID No. 50 |
| C1 | IYQRPVEFPNLPLKPR | SEQ ID No. 51 |
| C2 | YQRPVEFPNLPLKPR | SEQ ID No. 52 |
| C3 | QRPVEFPNLPLKPR | SEQ ID No. 53 |
| C4 | RPVEFPNLPLKPR | SEQ ID No. 54 |
| C5 | PVEFPNLPLKPR | SEQ ID No. 55 |
| C6 | VEFPNLPLKPR | SEQ ID No. 56 |
| C7 | EFPNLPLKPR | SEQ ID No. 57 |
| C8 | PMDDIYQRPVEFPNLPLKP | SEQ ID No. 58 |
| C9 | PMDDIYQRPVEFPNLPLK | SEQ ID No. 59 |
| C10 | PMDDIYQRPVEFPNLPL | SEQ ID No. 60 |
| C12 | PMDDIYQRPVEFPNLP | SEQ ID No. 61 |
| D1 | PMDDIYQRPVEFPNL | SEQ ID No. 62 |
| D2 | PMDDIYQRPVEFPN | SEQ ID No. 63 |
| D3 | PMDDIYQRPVEFP | SEQ ID No. 64 |
| D4 | PMDDIYQRPVE | SEQ ID No. 65 |
| D5 | PMDDIYQRPV | SEQ ID No. 66 |

Um die wichtigen Reste zu identifizieren wurden zusätzlich 20 Peptide in synthetisiert, bei denen jeweils eine Aminosäure durch Alanin ersetzt wurde.

| | | |
|---|---|---|
| A1 | PMDDIYQRPVEFPNLPLKPR | SEQ ID No. 25 |
| A2 | PMDDIYQRPVEFPNLPLKPA | SEQ ID No. 67 |
| A3 | PMDDIYQRPVEFPNLPLKAR | SEQ ID No. 68 |
| A4 | PMDDIYQRPVEFPNLPLAPR | SEQ ID No. 69 |
| A5 | PMDDIYQRPVEFPNLPAKPR | SEQ ID No. 70 |
| A6 | PMDDIYQRPVEFPNLALKPR | SEQ ID No. 71 |
| A7 | PMDDIYQRPVEFPNAPLKPR | SEQ ID No. 72 |
| A8 | PMDDIYQRPVEFPALPLKPR | SEQ ID No. 73 |
| A9 | PMDDIYQRPVEFANLPLKPR | SEQ ID No. 74 |
| A10 | PMDDIYQRPVEAPNLPLKPR | SEQ ID No. 75 |
| A11 | PMDDIYQRPVAFPNLPLKPR | SEQ ID No. 76 |
| A12 | PMDDIYQRPAEFPNLPLKPR | SEQ ID No. 77 |
| B1 | PMDDIYQRAVEFPNLPLKPR | SEQ ID No. 78 |
| B2 | PMDDIYQAPVEFPNLPLKPR | SEQ ID No. 79 |
| B3 | PMDDIYARPVEFPNLPLKPR | SEQ ID No. 80 |
| B4 | PMDDIAQRPVEFPNLPLKPR | SEQ ID No. 81 |
| B5 | PMDDAYQRPVEFPNLPLKPR | SEQ ID No. 82 |
| B6 | PMDAIYQRPVEFPNLPLKPR | SEQ ID No. 83 |
| B7 | PMADIYQRPVEFPNLPLKPR | SEQ ID No. 84 |
| B8 | PADDIYQRPVEFPNLPLKPR | SEQ ID No. 85 |
| B9 | AMDDIYQRPVEFPNLPLKPR | SEQ ID No. 86 |

Diese ungereinigten Peptide (~7,27 µM Endkonzentration im Assay) wurden im oben beschriebenen Berichrom Assay auf ihre inhibitorische Aktivität untersucht. Dabei ergaben sich folgende, in Abb. 7 gezeigte Messwerte.

Zur weiteren Überprüfung der Ergebnisse wurden die beiden Sequenzen MDDIYQRPVEFPNLPL (SEQ ID No. 87) 16mer) und DDIYQRPVEFPNLP (SEQ ID No. 88), 14mer) synthetisiert und gereinigt. Die Werte für die Ausgangssequenz (SEQ ID No. 25) wurden mit gereinigtem Peptid zum Vergleich gemessen. Damit konnte dann die inhibitorische Aktivität (IC₅₀) im oben erwähnten Berichrom^{®}-Test bestimmt werden:
- (SEQ ID No. 87) IC₅₀ = 19 µM
- (SEQ ID No. 88) IC₅₀ = ~280 µM

### Beispiel 4: Expression und Reinigung von durch Austausch von Cystein-Resten modifizierten Trideginen aus Escherichia coli

Diese modifizierten Tridegine wurden durch gezielte Mutagenese der im wildtyp Tridegin enthaltenen Cysteine erzeugt. Das Ziel dieser Mutagenese war der schrittweise Austausch der Cysteinreste, die zur Ausbildung von intermolekularen Disulfidbrücken geeignet sind. Die Neigung zur Bildung von Disulfidbrücken und die damit einhergehende Aggregation des Endproduktes wurde durch entsprechende chromatographischen Analysen des originären Transglutaminase-Inhibitor (SEQ ID No.1) in An- bzw. Abwesenheit von reduzierenden Agenzien (z.B. Mercaptoethanol, DTT) nachgewiesen. So zeigte das aufgereinigte, rekombinante Tridegin Polypeptid bei einem Gelfiltrationslauf (auf einer HiPrep 26/60 Sephacryl-S200 HR Säule der Fa. Amersham-Pharmacia; 20mM Natriumphosphat pH 8,0, 300mM NaCl; 1 ml/min) etwa einen Anteil von 80% multimeren, hochmolekularen Aggregaten. Die Anteil der Aggregate war signifikant geringer (< 20 %) wenn das rekombinante Tridegin unter reduzierenden Bedingungen in einem Gelfiltrationslauf in 1.5 mM DTT, 20mM Natriumphosphat pH 8,0, 300mM NaCl aufgetrennt wurde.

Die Mutagenese wurde mit den QuikChange Reagenzien (der Fa. Stratagene) laut Anleitung des Herstellers durchgeführt. Die Oligonukleotide SEQ ID No. 41 bis SEQ ID No. 46 und die jeweiligen revers-komplementären Sequenzen wurden für die Mutagenese verwendet. Die DNA-Sequenzen der erhaltenen Mutanten wurden durch Sequenzierung überprüft.

Die jeweilige codierende Sequenz im Plasmid pET22b wurde nach gängigen Verfahren in den *Escherichia coli* -Expressionsstamm Origami^{®} B(DE3) (der Fa. Novagen) transferiert und in LB-Flüssigmedium wie oben beschrieben kultiviert. Die Expression, Reinigung und Aktivitätsbestimmung erfolgte wie in Beispiel 1 genannt. Folgende inhibitorische Aktivitäten wurden für die einzelnen Mutanten gemessen (siehe Tabelle 4). Die Bezeichnung der Mutanten erfolgte nach dem Schema XnY.

Dabei bezeichnet X die Aminosäure, die durch Mutagenese verändert wurde, n definiert die Position dieser Aminosäure in der Polypeptidkette, und Y bezeichnet die nach der Mutagenese vorhandene Aminosäure.

**Tabelle 4: Inhibitorische Wirkung von durch Austausch von Cystein-Resten modifizierten Trideginen auf den Faktor XIIIa im Berichom^{®}-Assay.**

| Cys-Variante | Relative inhibitorische Wirkung (%) | Oligonukleotid | SEQ ID No. |
|---|---|---|---|
| zum Vergleich SEQ ID No. 1 | 100 | | |
| C06A | 72 | | 41 |
| C18A | 93 | | 42 |
| C20A | 63 | | 43 |
| | | | |
| C26A | 82 | | 44 |
| C32A | 86 | | 45 |
| C26A;+C 38A | 80 | | |
| C38A | | | 46 |

Die relative inhibitorische Wirkung (%) wurde bei einer Endonzentration der Variante von 5,45 µM bestimmt. Die inhibitorische Wirkung des rekombinanten wildtyp Tridegin Polypeptides wurde auf 100 % (bei 5,45 µM) normiert. Die zur Erzeugung der oben genannten Varianten verwendeten Oligonukleotide sind in der Tabelle ebenfalls angegeben.

### Beispiel 5: Verbesserung der fibrinolytischen Aktivität von Gewebe-Plasminogen Aktivator (tPA) und Urokinases in Anwesenheit von rekombinantem Tridegin oder einem Fragment von Tridegin.

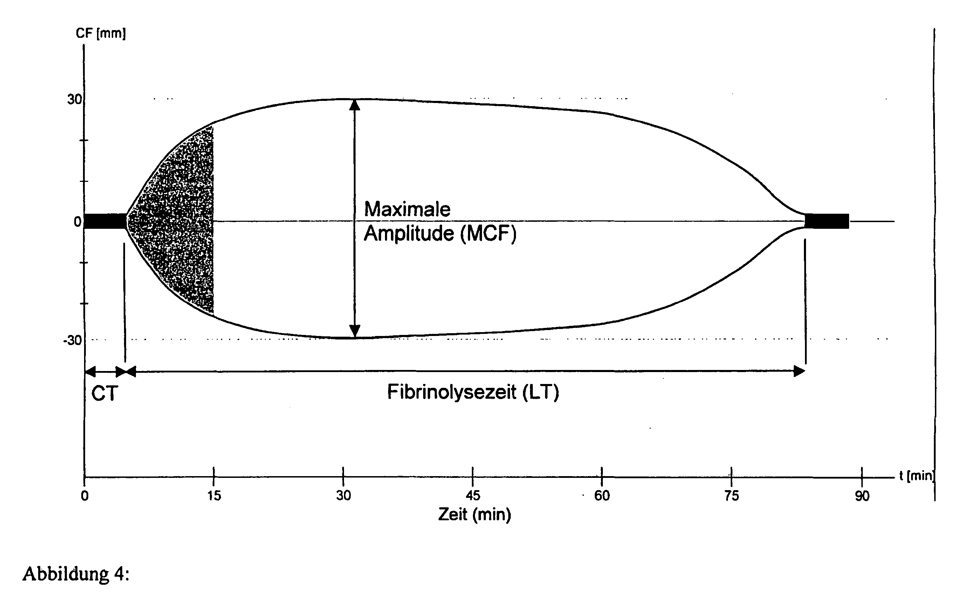

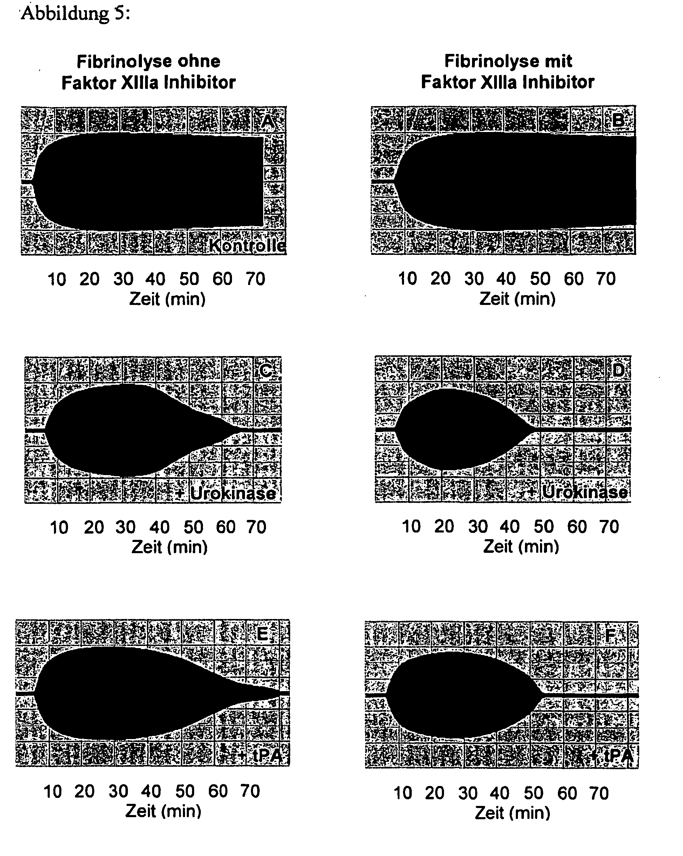

Um das therapeutische Potential der erfindungsgemäßen Polypeptide aufzuzeigen wurde die Blutgerinnung und Fibrinolyse in Anwesenheit von rekombinantem Tridegin Polypeptid aus *E. coli* (SEQ ID NO: 1, Abbildung 5) oder Tridegin Polypeptid aus Pichia Pastoris (SEQ ID NO: 91, Abbildung 11) oder Fragmenten von Tridegin (SEQ ID NO: 25, Abbildung 10) in Vollblut gemessen. Dazu wurden sogenannte Thrombelastogramme aufgezeichnet. Die Thrombelastographie ist ein gängiges Verfahren zur Messung von Gerinnung und Fibrinolyse. Dabei wird die Änderung der Viskosität des Blutes durch die Änderung des Drehwiderstandes eines im Blut befindlichen Stempels gemessen (Calatzis et al., 2000). Die Abbildung 4 zeigt die typischen Phasen der Gerinnung und Fibrinolyse in einem Thrombelastogramm. Thrombelastogramme quantifizieren wichtige Parameter der Hämostase:
- Gerinnungszeit (Zeit zwischen Initiierung der Gerinnung bis zum Beginn einer messbaren Änderung der Blut-Viskosität, Clotting time, CT)
- Stabilität des Thrombus (Maximale Amplitude, Maximum Clot Firmness, MCF)-
- Zeit der Fibrinolyse (Zeit zwischen dem Beginn einer messbaren Änderung der Blut-Viskosität und Erreichen des Ausgangswertes vor der Gerinnung, Lysis-time, LT).

Für die dargestellten Messungen wurde das ROTEG®-Gerät der Fa. Pentapharm GmbH, München verwendet. Abbildung 5 und 6 zeigen die Thrombelastogramme von Citrat-Vollblut nach Zugabe von Ca ²⁺ (Starteg-Reagenz, Fa. Pentapharm GmbH) und Thromboplastin-Phospholipid (Integ-Reagenz, Fa. Pentapharm GmbH). Diese Reagenzien dienen zur Induktion der Koagulation.

Um die erfindungsgemäße synergistische Wirkung von herkömmlichen, in der Thrombose-Therapie verwendeten, fibrinolytischen Agenzien mit rekombinantem Tridegin Polypeptid und modifizierten Trideginen zu zeigen, wurden verschiedene Versuche durchgeführt. Die Thrombelastogramme zeigen, dass das rekombinante Tridegin Polypeptid und ein modifiziertes Tridegin die Fibrinolyse durch die als Beispiel gewählten fibrinolytischen Agenzien Gewebe-Plasminogen Aktivator (tPA) und Urokinase beschleunigt und verbessert. Dies wird durch
a) die geringere Amplitude (geringere Stabilität des Thrombus) und
b) die beschleunigte Fibrinolyse deutlich.

Aus der Verlängerung der Gerinnungszeit (CT) von 190 Sekunden in Abwesenheit eines Transglutaminase Inhibitors zu 380 Sekunden in Anwesenheit des rekombinanten wildtyp Tridegin Polypeptides aus E. *coli* (SEQ ID NO: 1) oder eines modifizierten Tridegins wird außerdem ersichtlich, dass das rekombinante Tridegin Polypeptid und ein modifiziertes Tridegin zusätzlich die Blutgerinnung hemmt (vgl. Abb 5 A und B).

### Beispiel 6: Expression und Reinigung von rekombinantem Tridegin Polypeptid (codiert von SEQ ID No.: 90) aus Pichia pastoris.

Das Expressionsplasmid TrideginpPICZαA (auf Grundlage des Expressionsvektors pPICZαA, Invitrogen), das die codierende Sequenz für das rekombinante Tridegin enthält, ist in Abbildung 8 dargestellt.

Durch die Fusion mit dem Alpha-Faktor Signalpeptid kann das Tridegin in das Kulturmedium sekretiert werden. Das Plasmid wurde nach gängigen Verfahren in die *Pichia pastoris* Stämme KM71H und SMD1168 transferiert. Klone mit stabil integrierter Tridegin-Sequenz wurden durch Selektion von Zeocin-resistenten Klonen und anschließendem Nachweis der Tridegin-DNA-Sequenz durch das gängige Verfahren der Polymerase Chain Reaction (PCR) ausgewählt.

Die erhaltenen Klone wurden als Einzelkolonie in 100 ml BMGH (1% Hefeextrakt; 2% Pepton; 100 mM K-Phosphat, pH 6; 1.34% Yeast Nitrogen Base; 4x10⁻⁵ % Biotin; 1% Glycerol) ca. 16 - 24 Stunden unter schütteln kultiviert (30°C). Die Zellen wurden abzentrifugiert (3000 x g, 5 min) und in 20 - 30 ml BMMH (1% Hefeextrakt; 2% Pepton; 100 mM K-Phosphat, pH 6; 1.34% Yeast Nitrogen Base; 4x10⁻⁵% Biotin; 0,5 % Methanol) resuspendiert und wieder bei 30°C unter Schütteln inkubiert. Nach 24 Stunden wurde Methanol (0.5 % Endkonzentration) zugegeben. Nach weiteren 24 Stunden wurden die Zellen wie oben beschrieben abzentrifugiert. Der Kulturüberstand wurde direkt verarbeitet oder bei -70°C gelagert.

Das Tridegin Polypeptid konnte durch SDS-Polyacrylamid Gel-Elektrophorese und Coomassie Brilliant Blue Färbung nachgewiesen werden. Die korrekte Prozessierung des Signalpeptides konnte durch N-terminale Sequenzierung (Edman Abbau, Toplab GmbH) des Polypeptids bestätigt werden. Allerdings konnte das vollständig exprimierte Tridegin Polypeptid mit den C-terminalen 6 Histidin-Resten nur in geringer Menge mit Hilfe eines gängigen Western-Blotting Verfahren (Antikörper gegen 5 konsekutive Histidin-Reste, Quiagen AG) nachgewiesen werden. Die fehlende C-terminale Proteinsequenz GluGluSerLeuGluHisHisHis-HisHisHis konnte mittels Massenspektroskopie (MALDI, Toplab GmbH) ermittelt werden.

Das Hauptprodukt, ein Tridegin Polypeptid ohne die 11 C-terminalen Reste, wurde durch folgendes Verfahren gereinigt. Der Kulturüberstand wurde mit 10 g (NH₄)₂SO₄ pro 25 ml Kulturüberstand versetzt. Das entstehende Präzipitat wurde abzentrifugiert und in 20 mM CHES, pH 9 gelöst und anschließend gegen 20 mM CHES, pH9 dialysiert. Die Probe wurde dann auf eine Sepharose Q (25 ml)- oder Resource Q (1 ml)-Ionenaustauschersäule (beides Amersham Biosciences) geladen (Flussrate 1-4 ml/min). Die Elution erfolgte durch einen Gradienten von 20 mM CHES, pH9 gegen 20 mM CHES, 1M NaCl, pH 9. Die während der Elution gesammelten Fraktionen wurden mittels SDS-Polyacrylamid Gel-Elektrophorese aufgetrennt und mit Coomassie Brilliant Blue gefärbt. Dadurch konnten Tridegin Polypeptid enthaltene Fraktionen identifiziert werden. Diese wurden vereinigt und gegen PBS (0,2 g/l KCI, 0,2 g/l KH₂PO₄, 8 g/l NaCl, 1,15 g/l Na₂HPO₄, pH 7,2) dialysiert und durch Ultrafiltration (Centricon YM-3, Amicon) eingeengt. Abbildung 9 zeigt eine FXIIIa Hemmkurve mit den gewonnenen Tridegin-Proben aus *Pichia pastoris*. Zur Bestimmung der Aktivität wurde der oben beschriebene Berichrom Assay verwendet.

### Beispiel 7: Expression und Reinigung von modifiziertem rekombinantem Tridegine Polypeptid (codiert von SEQ ID NO: 91) aus Pichia pastoris.

Dem in Beispiel 6 exprimierten und gereinigten Tridegin Polypeptid fehlen 6 C-terminalen Histidine. Vermutlich wurden diese durch eine Protease abgespalten. Deshalb wurde die codierende DNA Sequenz durch ein gängiges gerichtetes Mutagenese-Verfahren verändert um den Protease-Verdau des codierten Tridegin Polypeptides zu inhibieren. Dabei wurde das Expressionsplasmid pPICZalphaA-TrideginR66L generiert (Tridegin-Sequenz SEQ ID 91). In der Tridegin Polypeptid-Sequenz wurde ein Arginin-Rest am C-Terminus gegen Leucin ausgetauscht.

Das Expressionsplasmid wurde wie in Beispiel 6 beschrieben zur Expression von Tridegin Polypeptid genutzt. Dabei zeigte sich durch Anwendung des oben beschriebenen Western-Blotting Verfahren, dass die komplette Protein-Sequenz mit den C-terminalen Histidinen in überraschend erhöhter Ausbeute detektiert werden konnte.

Dieses Produkt konnte durch folgendes Verfahren gereinigt werden.

Der Kulturüberstand wurde mit 1 M Na-Phosphat, pH 8 versetzt bis ein pH von 7,4 erzielt wurde. Der Kulturüberstand wurde dann mit Puffer A (50 mM NaH₂PO₄ pH 8, 300 mM NaCl, 10 mM Imidazol) 1:1 verdünnt und wie in Beispiel 1 beschrieben über eine Nickel-NTA Säule (Quiagen) geleitet. Die Säule wurde dann mit dem Puffer A gewaschen und mit Elutionspuffer (50 mM NaH₂PO₄, 300 mM NaCl, 250 mM Imidazol pH 8) eluiert. Fraktionen wurden gesammelt und mit SDS-PAGE auf die Anwesenheit des Transglutaminase Inhibitors geprüft. Fraktionen, die das Tridegin Polypeptid enthalten wurden vereinigt und gegen PBS (0,2 g/l KCI, 0,2 g/l KH₂PO₄, 8 g/l NaCl, 1,15 g/l Na₂HPO₄, pH 7,2) dialysiert. Abbildung 9 zeigt eine FXIIIa Hemmkurve mit diesen so gewonnen Tridegin-Proben aus *Pichia pastoris*. Zur Bestimmung der Aktivität wurde der oben beschriebene Berichrom^{®} Assay verwendet.

### Beispiel 8

Der Einfluss der vom Tridegin Polypeptid abgeleiteten Peptide auf die Blutgerinnung und Fibrinolyse wurde auch gemessen (Abbildung 10). Die Peptide (SEQ ID 25 und SEQ ID 88) wurden in PBS gelöst und verdünnt. Für die in Abbildung 10 gezeigten Messungen wurde Vollblut (24 h bei 4°C gelagert) verwendet

Der Einfluss von rekombinantem, gereinigtem Tridegin-Polypeptid aus *Pichia pastoris* auf die Blutgerinnung und Fibrinolyse ist in Abbildung 11 gezeigt. Für diese Messungen wurde das Tridegin Polypeptid (Tridegin R66L, codiert von SEQ ID No. 91) verwendet. Es vermindert die maximale Amplitude der Thrombelastogramme (d.h die Stabilität des Gerinnsels, MCF) und verringert zusätzlich die Fibrinolysezeit (LT) in Anwesenheit von tPA oder Urokinase.

## Patentansprüche

1. Polypeptid gemäß SEQ ID NO: 1 **dadurch gekennzeichnet, daß** es mindestens eine Deletion des N- und/oder des C-Terminus enthält, wobei das verbleibende Polypeptid mindestens die Aminosäuresequenz DDIYQRXVXFPXLPL (SEQ ID NO: 89) enthält.

2. Polypeptid nach Anspruch 1, wobei das Polypeptid eine Substitution mindestens einer der folgenden Aminosäuren - Lys2, Lys7, His10, Gly12, Leu24, Tyr31, Phe34, Arg39, Ile45, Met48, Pro55, Asn60, Pro65 - durch eine andere Aminosäure enthält.

3. Polypeptid nach Anspruch 1 oder 2, wobei das Polypeptid eine Substitution mindestens eines Cysteins durch eine andere Aminosäure enthält.

4. Verbindung enthaltend mindestens ein Polypeptid nach einem der Ansprüche 1 bis 3
- und eine kovalenten Verknüpfung mit Polyethylenglykol,
- in einem Fusionsprotein mit einem Tag, der der Reinigung des Fusionsproteins dient und/oder
- in einem Fusionsprotein mit einer Peptid- oder Polypeptidsequenz, die die Sekretion des Fusionsproteine aus einem Wirt vermittelt.

5. Verbindung nach Anspruch 4, wobei der Tag mindestens fünf aufeinanderfolgende Histidine enthält.

6. Nukleinsäure kodierend für ein Polypeptid nach mindestens einem der Ansprüche 1 bis 3 oder ein Fusionsproteins wie definiert in Anspruch 4 oder 5.

7. Nukleinsäure nach Anspruch 6 als Teil eines Vektors, insbesondere als Teil eines prokaryontischen oder eukaryontischen Expressionsvektors.

8. Verfahren zur Herstellung eines Polypeptides nach mindestens einem der Ansprüche 1 bis 3 oder einer Verbindung nach Anspruch 4 oder 5, **dadurch gekennzeichnet, daß** es sich um ein gentechnisches oder peptidchemisches Verfahren handelt.

9. Verwendung eines Polypeptides nach mindestens einem der Ansprüche 1 bis 3 und/oder einer Verbindung nach Anspruch 4 oder 5 als Transglutaminaseinhibitor.

10. Verwendung eines Polypeptides nach mindestens einem der Ansprüche 1 bis 3 und/oder einer Verbindung nach Anspruch 4 oder 5 zur Herstellung eines Arzneimittels zur Prävention und Therapie von Thrombosen.

11. Arzneimittel enthaltend
- ein Polypeptid nach mindestens einem der Ansprüche 1 bis 3 und/oder eine Verbindung nach Anspruch 4 oder 5; und
- mindestens einen galenischen Hilfsstoff.

12. Kombinationspräparat enthaltend
- ein Polypeptid nach mindestens einem der Ansprüche 1 bis 3 und/oder eine Verbindung nach Anspruch 4 oder 5; und
- mindestens einen weiteren pharmazeutischen Wirkstoff.

13. Kombinationspräparat nach Anspruch 12, **dadurch gekennzeichnet, daß** der weitere Wirkstoff ein Antikoagulanz, vorzugsweise Inhibitoren von Thrombin, Faktor Xa und/oder Inhibitoren der Blutplättchen-Aggregation, ist.

14. Kombinationspräparat enthaltend
- ein Polypeptid nach mindestens einem der Ansprüche 1 bis 3 und/oder eine Verbindung nach Anspruch 4 oder 5; und
- gegebenenfalls einen weiteren pharmazeutischen Wirkstoff in beliebiger Kombination mit Acetylsalicylsäure, Heparin, niedermolekularem Heparin, Heparinoid, Hirudin, Bivalirudin, Melagatran, Abciximab, Eptifibabide, Gewebe Plasminogen Aktivator (tPA), Streptokinase, Staphylokinase, Urokinase, Eminase, Hementin und/oder Plasmin.

## Claims

1. A polypeptide as depicted in SEQ ID NO: 1, **characterized in that** it contains at least one deletion of the N and/or C terminus, with the remaining polypeptide containing at least the amino acid sequence DDIYQRXVXFPXLPL (SEQ ID NO. 89).

2. The polypeptide according to claim 1, wherein the polypeptide contains one replacement of at least one of the following amino acids - Lys2, Lys7, His10, Gly12, Leu24, Tyr31, Phe34, Arg39, Ile45, Met48, Pro55, Asn60, Pro65 - with another amino acid.

3. The polypeptide according to claim 1 or 2, wherein the polypeptide contains a replacement of at least one cysteine with another amino acid.

4. A compound containing at least one polypeptide as claimed in any of claims 1 to 3
- and a covalent linkage with polyethylene glycol,
- in a fusion protein with a tag, which is used for purifying the fusion protein, and/or
- in a fusion protein with a peptide or polypeptide sequence, which mediates the secretion of the fusion protein from a host.

5. A compound as claimed in claim 4, wherein the tag contains at least five consecutive histidines.

6. A nucleic acid, which encodes a polypeptide as defined in at least one of claims 1 to 3 or a fusionprotein as defined in claim 4 or 5.

7. A nucleic acid as claimed in claim 6 as part of a vector, in particular as part of a prokaryotic or eukaryotic expression vector.

8. A method for preparing a polypeptide as claimed in at least one of claims 1 to 3 or a compound according to claim 4 or 5, **characterized in that** it is a recombinant method or a method of peptide chemistry.

9. The use of a polypeptide as claimed in at least one of claims 1 to 3 and/or of a compound according to claim 4 or 5 as a transglutaminase inhibitor.

10. The use of a polypeptide as claimed in at least one of claims 1 to 3 and/or of a compound according to claim 4 or 5 for the manufacture of a pharmaceutical for the prevention and therapy of thromboses.

11. A pharmaceutical containing
- a polypeptide as claimed in at least one of claims 1 to 3 and/or a compound according to claim 4 or 5; and
- at least one galenic adjuvant.

12. A combination preparation containing
- a polypeptide as claimed in at least one of claims 1 to 3 and/or a compound according to claim 4 or 5; and
- at least one additional pharmaceutical active compound.

13. The combination preparation as claimed in claim 12, **characterized in that** the additional active compound is an anticoagulant, preferably inhibitors of thrombin, factor Xa and/or inhibitors of blood platelet aggregation.

14. A combination preparation
- containing a polypeptide as claimed in at least one of claims 1 to 3 and/or a compound according to claim 4 or 5 and,
- optionally, an additional pharmaceutical active compound in arbitrary combination with acetylsalicylic acid, heparin, low molecular weight heparin, heparinoid, hirudin, bivalirudin, melagatran, abciximab, eptifibabide, tissue plasminogen activator (tPA), streptokinase, staphylokinase, urokinase, eminase, hementin and/or plasmin.

## Revendications

1. Polypeptide selon SEQ ID NO : 1 **caractérisé en ce qu'**il contient au moins une délétion du terminus N et/ou C, le polypeptide restant contenant au moins une séquence d'acide aminé DDIYQRXVXFPXLPL (SEQ ID NO : 89).

2. Polypeptide selon la revendication 1, dans lequel polypeptide au moins l'un des acides aminés suivants - Lys2, Lys7, His10, Gly12, Leu24, Tyr31, Phe34, Arg39, Iule45, Met48, Pro55, Asn60, Pro65 - est substitué par un autre acide aminé.

3. Polypeptide selon la revendication 1 ou 2, le polypeptide contient au moins une cystéine substituée par un autre acide aminé.

4. Combinaison contenant au moins un polypeptide selon l'une quelconque des revendications 1 à 3 et une liaison covalente avec du polyéthylène glycol,
- dans une protéine de fusion avec un marqueur, qui sert au nettoyage de la protéine de fusion
et/ou
- dans une protéine de fusion avec un peptide ou une séquence de polypeptide, qui transmet la sécrétion de la protéine de fusion à partir d'un hôte.

5. Combinaison selon la revendication 4, le marqueur contenant au moins cinq histidines qui se suivent.

6. Acide nucléique codant pour un polypeptide selon au moins l'une quelconque des revendications 1 à 3 ou une protéine de fusion telle que définie dans la revendication 4 ou 5.

7. Acide nucléique selon la revendication 6 en tant que partie d'un vecteur, en particulier en tant que partie d'un vecteur d'expression procaryote ou eucaryote.

8. Procédé pour la fabrication d'un polypeptide selon l'une quelconque des revendications 1 à 3 ou une combinaison selon la revendication 4 ou 5, **caractérisé en ce qu'**il s'agit d'un procédé technique génique ou peptide chimique.

9. Utilisation d'un polypeptide selon au moins l'une quelconque des revendications 1 à 3 et/ou une combinaison selon la revendication 4 ou 5 en tant qu'inhibiteur transglutaminase.

10. Utilisation d'un polypeptide selon au moins l'une quelconque des revendications 1 à 3 et/ou une combinaison selon la revendication 4 ou 5 pour la fabrication d'un médicament pour la prévention et le traitement de thromboses.

11. Médicaments contenant
- un polypeptide selon au moins l'une quelconque des revendications 1 à 3 et/ou une combinaison selon la revendication 4 ou 5 ; et
- au moins un agent adjuvant galénique.

12. Préparation d'une combinaison contenant
- un polypeptide selon au moins l'une quelconque des revendications 1 à 3 et/ou une combinaison selon la revendication 4 ou 5 ; et
- au moins un autre agent pharmaceutiquement actif.

13. Préparation d'une combinaison selon la revendication 12, **caractérisée en ce que** l'autre agent actif est un anticoagulant, de préférence inhibiteurs de thrombine, facteur Xa et/ou inhibiteurs d'agglomération de plaquettes sanguines.

14. Préparation d'une combinaison contenant
- un polypeptide selon l'une quelconque des revendications 1 à 3 et/ou une combinaison selon la revendication 4 ou 5 ; et
- le cas échéant un autre agent pharmaceutiquement actif dans une combinaison quelconque avec de l'acide acétylsalicylique, de l'héparine, de l'héparine de faible poids moléculaire, de l'héparinoïde, de l'hirudine, de la bivalirudine, du melagatran, de l'abciximab, de leptifibatide, un activateur tissulaire du plasminogène (tPA), de la streptokinase, de la staphylokinase, de l'urokinase, de l'éminase, de l'hémentine et/ou de la plasmine.
